# EUROPÄISCHE PATENTSCHRIFT

(11) **EP 0 457 182 B1**
(45) Veröffentlichungstag und Bekanntmachung des Hinweises auf die Patenterteilung: **26.02.1997**
(21) Anmeldenummer: 91107496.1
(22) Anmeldetag: 08.05.1991
(51) Int. Cl.: G01N 31/22, C07C 205/24, C07C 205/36, C07C 255/66, C07C 317/32, G01N 33/84, C07C 39/26, C07C 39/27, C07C 205/37, C07C 245/10

(54) **Verfahren zur Bestimmung eines Ions mit erhöhter Empfindlichkeit, Verwendung hierfür geeigneter Substanzen und entsprechendes Mittel**
Method having increased sensitivity, for the determination of an ion, the use of suitable substances and corresponding means therefor
Méthode ayant une sensibilité augmentée pour la détermination d'un ion, utilisation de substances appropriées et moyens correspondants pour exécuter cette méthode

(30) Priorität: 15.05.1990 DE 4015592
(43) Veröffentlichungstag der Anmeldung: 21.11.1991
(73) Patentinhaber: BOEHRINGER MANNHEIM GMBH, 68298 Mannheim (DE)
(72) Erfinder: Rittersdorf, Walter, W-6800 Mannheim 31 (DE); Guethlein, Werner, W-6800 Mannheim 24 (DE); Thym, Detlef, W-6800 Mannheim 1 (DE); Vogel, Peter, W-6944 Hemsbach (DE)

(56) Entgegenhaltungen:
- EP-A- 0 343 441
- US-A- 3 800 051

## Beschreibung

Die Erfindung betrifft ein Verfahren zur Bestimmung eines Ions in einer wässrigen Flüssigkeit, insbesondere Körperflüssigkeit, wie Blut, Plasma, Serum oder Urin, bei dem das Ion in eine mit der wässrigen Flüssigkeit nicht mischbare Phase gelangt und ein dort anwesender pH-Indikator dadurch eine Farbänderung erfährt, welche zur Bestimmung des Ions herangezogen wird. Die Erfindung betrifft außerdem die Verwendung von Substanzen, die zur Erhöhung der Meßempfindlichkeit eines solchen Verfahrens führen. Weiter betrifft die Erfindung ein Mittel zur Bestimmung eines Ions in einer wässrigen Flüssigkeit, enthaltend ein Ionophor und einen pH-Indikator in einem mit Wasser nicht mischbaren Medium.

Zur Bestimmung von Ionen, speziell Alkali- und Erdalkali-Ionen in Lösungen sind zahlreiche Methoden bekannt. Die größte Bedeutung haben hierbei die Flammenphotometrie, die Atomabsorptionsspektrometrie und neuerdings auch ionenselektive Elektroden erlangt. Alle diese Verfahren bedürfen eines großen apparativen Aufwandes. Aus diesem Grunde ist man bemüht, nach Möglichkeiten zu suchen, diese Ionen nach für den Anwender einfach zu handhabenden Verfahren zu bestimmen. Solche Verfahren sind interessant für die schnelle Bestimmung von Natrium-Ionen bei der Meerwasserentsalzung, für die schnelle Bestimmung von Calcium-Ionen bei der Enthärtung von Wasser usw. Besonders wichtig sind schnell durchzuführende und einfach zu handhabende Methoden bei der Bestimmung von Natrium- und Kalium-Ionen in Körperflüssigkeiten, wie Blut, Plasma, Serum oder Urin, in der Labor- und Notfalldiagnostik von Herz- und Kreislauferkrankungen, Erkrankungen der Muskulatur, Nierenerkrankungen, Schockzuständen verschiedener Ursachen usw. Lithium-Bestimmungen werden beispielsweise bei der Kontrolle von Antidepressions-Therapien benötigt.

Zur einfachen Bestimmung von Ionen, insbesondere von Alkali-Ionen, die für die o. g. diagnostischen Fragestellungen besonders wichtig sind, sind zahlreiche Verfahren bekannt, die auf der Flüssig-Flüssig-Extraktion farbiger Anionen beruhen. Hierbei wird der wässrigen Lösung des Kations ein anionischer Farbstoff zugesetzt und danach wird mit einem mit Wasser nicht mischbaren Lösungsmittel, das ein Ionophor enthält, ausgeschüttelt. Das Ionophor, ein Komplexbildner für Alkali-Ionen, zieht mit dem Ion die ihm proportionale Menge des Farbstoffs in die organische Phase. Nach der Abtrennung der wässrigen Phase (mit dem überschüssigen Farbstoff) wird die organische Phase dann photometrisch ausgewertet.

Diese Methode ist zwar in der Naßchemie weit verbreitet, eignet sich aber nur wenig für die sogenannte Trockenchemie. Unter diesem Begriff versteht man Testträger, auch Schnelldiagnostica genannt, bei denen alle für die Nachweisreaktion benötigten Reagenzien im trockenen Zustand in oder auf einer oder mehreren Trägermatrices, wie saugfähigen oder quellbaren Materialien, vorliegen. Zur quantitativen Bestimmung einer Substanz wird eine flüssige Probe auf den Testträger aufgebracht und dort mit den zur Bestimmungsreaktion notwendigen Reagenzien in Kontakt gebracht. Als Maß für die zu bestimmende Substanz entsteht ein Signal, das gemessen werden kann. Wenn das Signal in einer Farbbildung oder Farbänderung besteht, kann diese visuell oder photometrisch, vorzugsweise remissionsphotometrisch ausgewertet werden.

Der Einfachheit des Testträgerprinzips widerspricht es, zur Bestimmung eines Ions der Probe einen Farbstoff zusetzen und dann dessen Überschuß entfernen zu müssen. Es nimmt daher nicht wunder, daß eine solche aus EP-A-0 041 175 bekannte Methode für einen Trockentest keine Bedeutung erlangt hat.

Besser geeignet für Testträger sind Bestimmungsverfahren für Kationen, die auf dem Prinzip einer sogenannten "heterogenen pH-Reaktion" basieren.

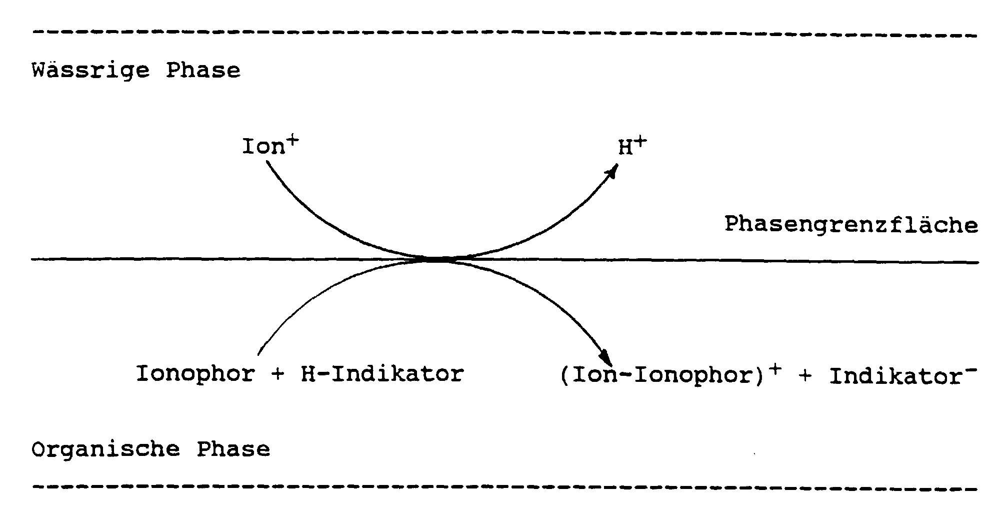

Es liegt hier ein Zwei-Phasensystem mit einer wässrigen und einer organischen Phase vor. In der organischen Phase ist ein für das nachzuweisende Kation spezifischer Ionophor und ein pH-Indikator gelöst. Beide chemischen Individuen können auch als Chromoionophor, d. h. über eine chemische Bindung zu einem einzigen Molekül vereint vorliegen. An der Grenzfläche beider Phasen wird das nachzuweisende Ion von dem Ionophor aufgenommen, in die organische Phase transportiert und liegt dort dann als Ion-Ionophor-Komplex vor. Zum Ladungsausgleich wird der ebenfalls in der organischen Phase befindliche pH-Indikator veranlaßt, ein Proton abzugeben, welches in die wässrige Phase übertritt. So entsteht eine zu der ursprünglich in der wässrigen Phase vorhandenen Konzentration des nachzuweisenden Ions proportionale Menge an farbigem Indikatoranion.

Für Flüssig-Flüssig-Extraktionen ist dieses Prinzip erstmalig bei E.S. Hyman, Biophysical Society Abstracts, 1971, 72a erwähnt und zwar mit Valinomycin als Ionophor und Tetrabromphenolphthaleinäthylester als pH-Indikator.

Beschreibungen mit Chromoionophoren finden sich beispielsweise in K.Ueno und M.Takagi, Studies in Physical and Theoretical Chemistry 27, 279-293, (1982) sowie H.Nakamura et al., Bunseki Kagaku 31, E131-E134 (1982).

In den genannten Veröffentlichungen wird als Analysen-Verfahren die Flüssig-Flüssig-Extraktion angewendet. Ausführungsformen von Schnelldiagnostica für Ionen, die auf diesem Prinzip basieren, sind schon verschiedentlich beschrieben worden. Sie unterscheiden sich im wesentlichen nur in ihrer Ausführungsform, d.h. wie die organische Phase in einer für Schnelldiagnostica brauchbaren Form realisiert ist. Generell besteht bei allen diesen Anmeldungen die organische Phase aus schwerflüchtigen, mit Wasser nicht mischbaren organischen Flüssigkeiten und/oder hydrophoben Polymeren. Liegen beide zusammen als feste Lösung vor, so spricht man auch von weichgemachten Kunststoffen.

In EP-A-0 125 555 ist eine Ausführungsform beschrieben, bei der die organische Phase als weichgemachter, unpolarer, nicht poröser Kunststoff-Film vorliegt, in dem Ionophor und Indikator gelöst sind. In EP-A-0 175 990 sind hydrophile, vorzugsweise wasserlösliche Partikel aus entsprechenden organischen Polymeren in eine organische Phase aus hydrophobem, filmbildendem, wasserunlöslichem Polymer eingebettet. In EP-A-0 125 554 ist eine Anwendungsform beschrieben, bei der die organische hydrophobe Phase in Form kleiner Tröpfchen in einer hydrophilen Matrix eingebettet ist. In EP-A-0 153 641 ist beschrieben, daß eine poröse Trägermatrix mit einer organischen hydrophoben Phase enthaltend Chromophor und Ionophor imprägniert wird. Papier wird als bevorzugte Trägermatrix beschrieben. In EP-A-0 141 647 ist die organische Phase als pigmentierter weichgemachter Kunststoff beschrieben, wobei auch hier Chromoionophore eingesetzt werden.

In der EP-A-0 343 441 werden fluorierte Bis-aryloxy-substituierte Alkene vorgestellt, die als Elektroisoliermittel verwendet werden können. Zur Herstellung solcher Verbindungen wird von Phenolderivaten ausgegangen, die mit einem Perhalogenalken umgesetzt werden.

Dem genannten Stand der Technik ist keine Lösung zu entnehmen für ein Problem, das allgemein bei der Auswertung von Ionen-Testen, die auf dem Prinzip der heterogenen pH-Reaktion beruhen, auftreten kann. Es ist dies das Problem, daß der Bereich der größten Meß-Empfindlichkeit nicht immer mit dem klinisch relevanten Konzentrationsbereich übereinstimmt. Dies wird beispielsweise dokumentiert durch folgendes: Der Normalbereich von Kalium in menschlichem Plasma liegt zwischen 3,5 und 5,5 mmol/l. Sowohl eine Überschreitung wie eine Unterschreitung der Normalwerte muß genau bestimmt werden können. Dies bedingt, daß der Bereich der größten Genauigkeit des Meßverfahrens diesen Konzentrationsbereich abdecken muß, um optimale Analysenergebnisse zu erzielen. Im Falle von Ionenbestimmungen nach dem Prinzip der heterogenen pH-Reaktion bedeutet dies, daß der Farbumschlag des pH-Indikators in dem Konzentrationsbereich des Ions, das bestimmt werden soll, eine möglichst gut meßbare Signaländerung ergeben muß. Im Falle reflektometrischer Messungen bedeutet dies, daß die Remissionsänderung im interessierenden Konzentrationsbereich des zu bestimmenden Ions möglichst groß sein muß. Das ist jedoch nicht immer der Fall.

Der Bereich des Farbumschlags eines pH-Indikators und damit der Bereich der größten Meßgenauigkeit sollte bei Ionenbestimmungen nach dem Prinzip der heterogenen pH-Reaktion eigentlich durch Zugabe einer Säure zur organischen Phase verschoben werden können. Man könnte sich hierfür folgenden Mechanismus vorstellen: Wird ein Elektrolytion vom Ionophor in die organische Phase gezogen, so sollte es nur von den Säurestärken und den Konzentrationen der dort vorliegenden Säure und des Indikators abhängen, wo das zur Aufrechterhaltung des Ladungsausgleichs in der wässrigen Phase notwendige Proton abgespalten wird. Wird es von der Säure abgespalten, dann erfolgt kein Farbumschlag. Wird es dagegen vom pH-Indikator abgespalten, wird ein Farbumschlag verursacht. Eine Säure, die stärker sauer ist als der Indikator, sollte also zunächst einmal Ionen-Äquivalente verbrauchen so daß erst im höheren Konzentrationsbereich des Ions der Indikator einen Farbumschlag bewirkt.

Versuche mit herkömmlichen lipophilen starken Säuren wie Halogencarbonsäuren, Halogen- und Nitrobenzolcarbonsäuren, Phosphorsäurediestern und dergleichen haben aber in der Praxis ergeben, daß diese Säuren bei Ionenbestimmungen nach dem Prinzip der heterogenen pH-Reaktion praktisch keine Wirkung haben, d.h. den Bereich der größten Empfindlichkeit so gut wie nicht beeinflussen.

Aufgabe der vorliegenden Erfindung war es deshalb, Substanzen bereitzustellen, die in Ionenbestimmungsverfahren die auf dem Prinzip der heterogenen pH-Reaktion basieren, sowie entsprechenden Mitteln eingesetzt werden können, um den Bereich der größten Empfindlichkeit des Verfahrens auf den Ionenkonzentrationsbereich abzustimmen, der untersucht werden soll.

Diese Aufgabe wird durch die in den Patentansprüchen charakterisierte Erfindung gelöst.

Gegenstand der Erfindung ist ein Verfahren zur Bestimmung eines Ions in einer wässrigen Flüssigkeit, bei dem das Ion durch einen Ionophor in eine mit der wässrigen Flüssigkeit nicht mischbare Phase gelangt und ein dort anwesender pH-Indikator dadurch eine Farbänderung erfährt, welche zur Bestimmung des Ions herangezogen wird und das dadurch gekennzeichnet ist, daß zur Erhöhung der Meßempfindlichkeit in der mit der wässrigen Flüssigkeit nicht mischbaren Phase eine Substanz aus der Gruppe der Verbindungen der allgemeinen Formel I 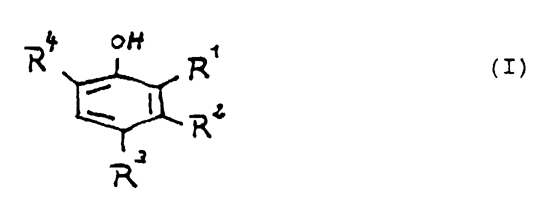 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 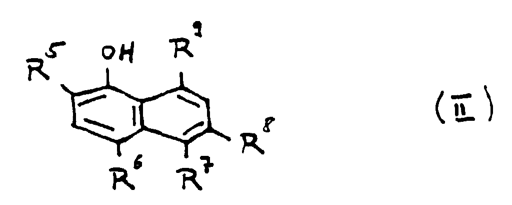 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können und
der allgemeinen Formel III 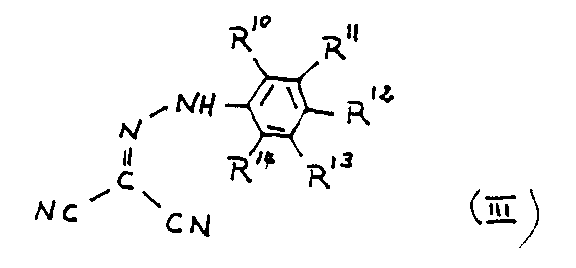 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
eingesetzt wird.

Gegenstand der Erfindung ist weiter die Verwendung einer Substanz aus der Gruppe der Verbindungen der allgemeinen Formel I 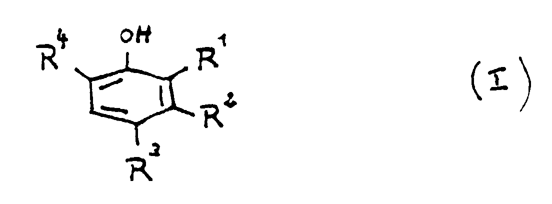 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 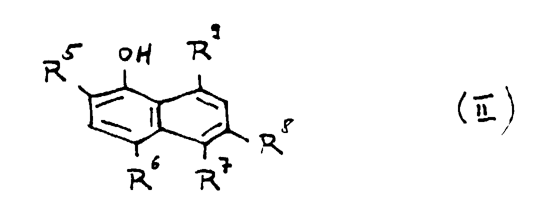 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können und
der allgemeinen Formel III 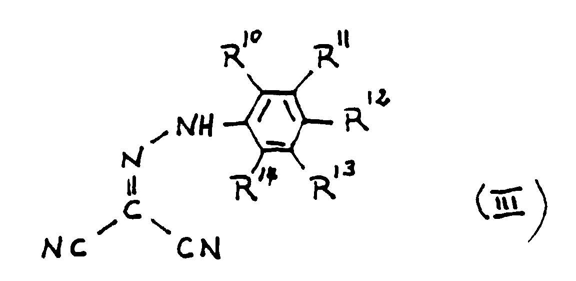 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten
zur Erhöhung der Meßempfindlichkeit bei Verfahren zur Bestimmung von Ionen, bei denen die Ionen durch Ionophore in eine mit der wässrigen Flüssigkeit nicht mischbare Phase gelangen und ein dort auwesender pH-Indikator dadurch eine Farbänderung erfährt, welche zur Bestimmung der Ionen herangezogen wird.

Weiter ist Gegenstand der Erfindung ein Mittel zur Bestimmung eines Ions in einer wässrigen Flüssigkeit, enthaltend einen Ionophor und einen pH-Indikator in einem mit Wasser nicht mischbaren Medium, dadurch gekennzeichnet, daß es zur Erhöhung der Meßempfindlichkeit eine Substanz aus der Gruppe der Verbindungen der allgemeinen Formel I 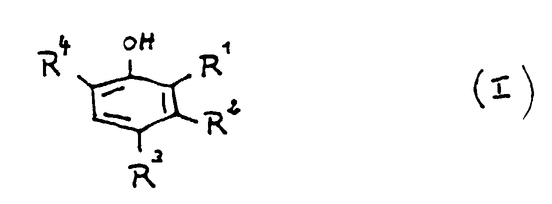 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 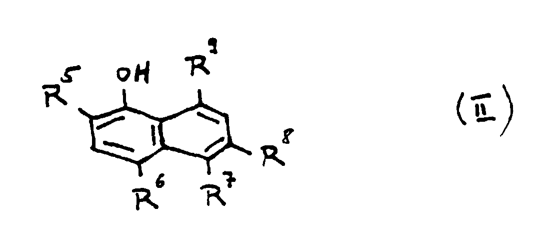 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können und
der allgemeinen Formel III 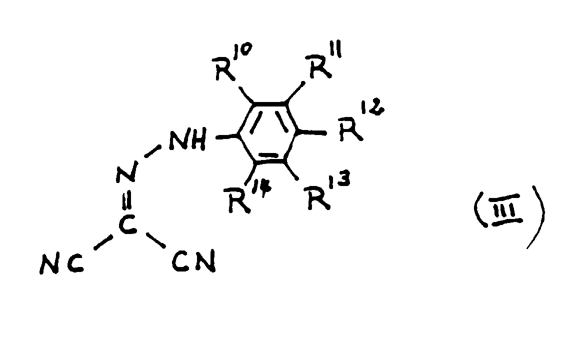 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
enthält.

Außerdem ist Gegenstand der Erfindung die Verwendung einer Substanz aus der Gruppe der Verbindungen der allgemeinen Formel I 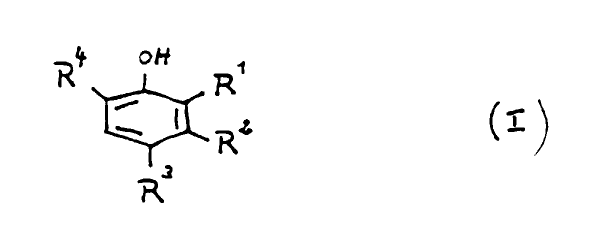 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 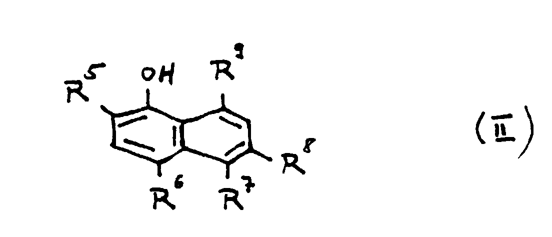 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können und
der allgemeinen Formel III 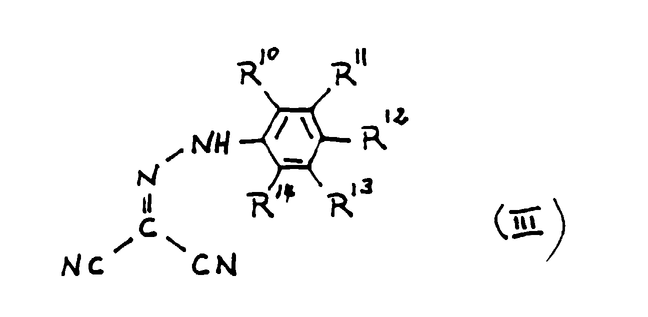 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
zur Herstellung eines Mittels zur Bestimmung eines Ions das einen Ionophor und einen pH-Indikator in einem mit Wasser nicht mischbaren Medium enthält.

Gegenstand ist auch eine Verbindung der allgemeinen Formel I' 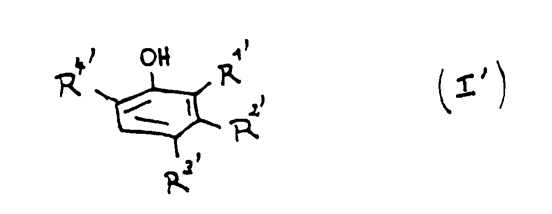 in der
R^{2'} einen Alkyl- oder Alkoxyrest darstellt und
R^{1'}, R^{3'} und R^{4'} gleich oder verschieden sind und jeweils eine Nitrogruppe, Halogen, eine Cyanogruppe, eine Alkylsulfonylgruppe oder eine mit Halogen substituierte Alkylgruppe bedeuten.

Weiter ist Gegenstand der Erfindung eine Verbindung der allgemeinen Formel II 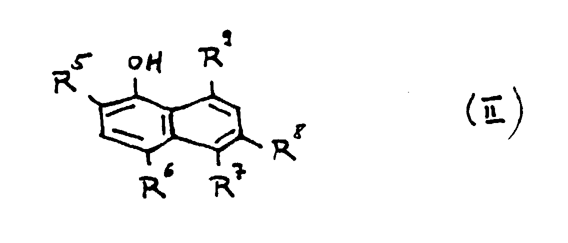 in der
- R⁷: einen Alkyl-, Alkoxy- oder Aralkylrest, insbesondere einen Alkoxyrest und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können.

Schließlich ist Gegenstand der Erfindung eine Verbindung aus der Gruppe der Substanzen
[(2,3,5,6-Tetrafluorphenyl)-hydrazono]propandinitril,
[(2-Trifluormethyl-4-nitrophenyl)-hydrazono]propandinitril,
[(2-Methansulfonyl-4-nitrophenyl)-hydrazono]propandinitril,
[(2,4-Dinitro-6-cyanophenyl)-hydrazono]propandinitril und
[(3,5-Di-{trifluormethyl}phenyl)-hydrazono]propandinitril.

Es hat sich überraschend gezeigt, daß es Säuren gibt, die bei Verfahren zur Bestimmung von Ionen auf Basis des Prinzips der heterogenen pH-Reaktion so wirken, daß sie zur Erhöhung der Meßempfindlichkeit in der mit der wässrigen Flüssigkeit nicht mischbaren Phase eingesetzt werden können. Es sind dies Phenole und Naphtole mit elektronenziehenden Resten, die zur Erhöhung der Lipophilie einen Alkylrest, einen Alkoxyrest oder einen Aralkylrest tragen. Außerdem sind auch Phenylhydrazonderivate des Mesoxalsäuredinitrils verwendbar, deren Phenylrest elektronenziehende Substituenten trägt.

Bewährt haben sich Verbindungen der allgemeinen Formel I, der allgemeinen Formel II und der allgemeinen Formel III in der Bedeutung wie vorstehend ausgeführt. Unter einem Alkylrest wie er als solches oder in einem Alkoxyrest in der Definition der Reste R¹ bis R⁴ bzw. R⁵ bis R⁹ vorliegt, wird ein Alkylrest mit 1 bis 30 Kohlenstoffatomen verstanden. Bevorzugt sind jedoch Reste mit 5 bis 30, vorzugsweise 10 bis 20 Kohlenstoffatomen. Ganz besonders bevorzugt sind solche mit 15 bis 18 Kohlenstoffatomen. Die Alkylgruppen in Alkylresten als solchen oder Alkoxyresten können geradkettig oder verzweigt, gesättigt oder ungesättigt sein.

Eine Alkylgruppe in der Bedeutung eines Aralkylrestes, einer Alkylsulfonylgruppe oder einer mit Halogen substituierten Alkylgruppe in der Definition der Reste R¹ bis R⁴, R⁵ bis R⁹ bzw. R¹⁰ bis R¹⁴ enthält 1 bis 4 Kohlenstoffatome. Die Methylgruppe ist besonders bevorzugt.

Halogen in der Bedeutung der Reste R¹ bis R¹⁴ bedeutet Fluor, Chlor, Brom oder Jod. Fluor, Chlor und Brom sind besonders bevorzugt. In der Bedeutung einer mit Halogen substituierten Alkylgruppe in der Definition der Reste R¹ bis R¹⁴ ist die Trifluormethylgruppe ganz besonders bevorzugt.

In einem Aralkylrest in der Definition der Reste R¹ bis R⁴ bzw. R⁵ bis R⁹ ist der aromatische Rest ein 6 bis 10 Kohlenstoffatome enthaltender Aromat. Vorzugsweise handelt es sich um einen Kohlenstoffaromaten. Ganz besonders bevorzugt ist der Phenylrest. Vorteilhafterweise ist der aromatische Teil eines Aralkylrestes durch einen oder mehrere elektronenziehende Reste, wie Nitro-, Halogen- oder Cyanoreste substituiert. Ein ganz besonders bevorzugter Aralkylrest ist ein Benzylrest, der einen oder mehrere elektronenziehende Reste trägt. Der Aralkylrest kann außer elektronenziehenden Resten auch noch eine Hydroxygruppe tragen. Ganz besonders bevorzugt ist nämlich ein Benzylrest mit einem oder mehreren elektronenziehenden Resten und einer Hydroxygruppe.

Bevorzugte Verbindungen der allgemeinen Formel I sind solche, bei denen R² einen Alkyl-, Alkoxy- oder Aralkylrest und R¹, R³ und R⁴ gleich oder verschieden sind und jeweils eine Nitrogruppe, Halogen, eine Cyanogruppe, eine Alkylsulfonylgruppe oder eine mit Halogen substituierte Alkylgruppe bedeuten. Hiervon eignen sich erfindungsgemäß solche Verbindungen besonders gut, die als Reste R¹, R³ und R⁴ gleiche oder verschiedene Reste aus der Gruppe Nitrorest, Halogen und Alkylsulfonylgruppe, tragen.

Vertreter der Verbindungen der allgemeinen Formel I, die erfindungsgemäß hervorragend wirken, sind beispielsweise 3-Pentadecyl-2,4,6-trinitrophenol oder Hexachlorophen (Bis-(2-hydroxy-3,5,6-trichlorphenyl)-methan).

Bevorzugte Verbindungen der allgemeinen Formel II sind solche, bei denen R⁷ einen Alkyl-, Alkoxy- oder Aralkylrest, insbesondere einen Alkoxyrest und R⁵, R⁶, R⁸ und R⁹ gleich oder verschieden sind und jeweils eine Nitrogruppe, Halogen, eine Cyanogruppe, eine Alkylsulfonylgruppe oder eine mit Halogen substituierte Alkylgruppe bedeuten. Hiervon eignen sich erfindungsgemäß solche Verbindungen besonders gut, die als Reste R⁵, R⁶, R⁸ und R⁹ gleiche oder verschiedene Reste aus der Gruppe Nitrorest, Halogen und Alkylsulfonylgruppe tragen. Von den vorstehend als bevorzugt genannten Verbindungen der allgemeinen Formel II sind diejenigen besonders bevorzugt, bei denen R⁶ eine Nitrogruppe ist.

Ein erfindungsgemäß hervorragend einsetzbarer Vertreter der Verbindungen der allgemeinen Formel II ist 2,4,6,8-Tetranitro-5-octadecyloxy-1-naphthol.

Erfindungsgemäß hervorragend einsetzbare Vertreter der Verbindungen der allgemeinen Formel III sind solche, bei denen mindestens zwei der Reste R¹⁰ bis R¹⁴ kein Wasserstoff sind.

Verbindungen der allgemeinen Formeln I, II und III eignen sich sehr gut dazu, um in Verfahren zur Bestimmung eines Ions in wässrigen Flüssigkeiten, insbesondere Körperflüssigkeiten, wie Blut, Plasma, Serum oder Urin, die auf dem Prinzip der "heterogenen pH-Reaktion", wie es eingangs beschrieben worden ist, beruhen, den Bereich der größten Empfindlichkeit des Verfahrens auf den Ionenkonzentrationsbereich abzustimmen, der untersucht werden soll. Die Verbindungen sind in wässrigen Lösungen nur schlecht, umso besser aber in organischen Lösungsmitteln löslich. Insofern können sie für den beabsichtigten Zweck in der organischen Phase gelöst und dort zum Einsatz gebracht werden. Es hat sich gezeigt, daß in Anwesenheit der erfindungsgemäßen Säuren die Ionenkonzentration über die Farbänderung eines pH-Indikators wesentlich genauer bestimmt werden kann als ohne Säure.

Als besonders bevorzugt hat sich in diesem Sinne die Kombination einer erfindungsgemäßen Säure mit einem pH-Indikator erwiesen, der der allgemeinen Formel IV 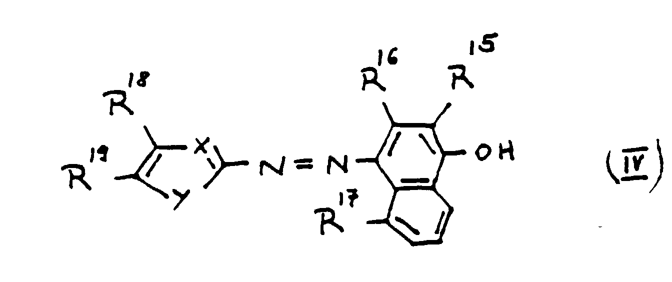 in der
- R¹⁵, R¹⁶, R¹⁷: gleich oder verschieden sind und jeweils Wasserstoff, eine Alkyl- oder Alkoxygruppe darstellen, wobei mindestens einer der Reste ein (C₈-C₃₀)-Alkyl- oder Alkoxyrest ist,
- R¹⁸: Wasserstoff oder eine Alkylgruppe,
- R¹⁹: eine Nitrogruppe, eine durch Halogen substituierte Alkylgruppe, eine Cyanogruppe, eine Sulfonamidgruppe oder eine Alkylsulfonylgruppe,
- X: Stickstoff oder den Rest CR²⁰ und
- Y: Schwefel oder den Rest CR²¹ = CR²²,

wobei R²⁰, R²¹, R²² gleich oder verschieden sind und jeweils Wasserstoff, Halogen, eine Nitrogruppe, eine Cyanogruppe, eine Alkyl- oder durch Halogen substituierte Alkylgruppe oder eine Alkylsulfonylgruppe
bedeuten, entspricht. Solche pH-Indikatoren sind in DE-A-4015591.9 beschrieben.

Unter einer Alkylgruppe in der Definition der Reste R¹⁵, R¹⁶, R¹⁷, R¹⁸, R²⁰, R²¹ und R²² wird ein Alkylrest mit 1 bis 30 Kohlenstoffatomen verstanden. Insbesondere für die Reste R¹⁸, R²⁰, R²¹ und R²² sind Alkylreste mit 1 bis 4 Kohlenstoffatomen, vor allem 1 bis 2 Kohlenstoffatomen, bevorzugt. Was die Reste R¹⁵, R¹⁶ und R¹⁷ anbelangt, so ist vorzugsweise nur einer der Reste ein Alkylrest mit 8 bis 30, vorzugsweise 10 bis 20 Kohlenstoffatomen. Sollten die übrigen Reste dieser Gruppe ebenfalls eine Alkylgruppe darstellen, so sind sie vorzugsweise ein Alkylrest mit 1 bis 4, insbesondere 1 bis 2 Kohlenstoffatomen. Alkylreste mit mehr als 2 Kohlenstoffatomen können geradkettig oder verzweigt sein. Außerdem kann der Alkylrest auch ungesättigt sein.

Unter einer durch Halogen substituierten Alkylgruppe in der Definition von R¹⁹, R²⁰, R²¹ und R²² wird ein durch Fluor, Chlor, Brom oder Jod substituierter Alkylrest mit 1 bis 4 Kohlenstoffatomen verstanden. Bevorzugt sind durch Fluor substituierte Alkylreste mit 1 bis 2 Kohlenstoffatomen. Besonders bevorzugt ist der Trifluormethylrest.

Eine Alkoxygruppe in der Definition der Reste R¹⁵, R¹⁶, R¹⁷ ist ein Alkoxyrest mit 8 bis 30, vorzugsweise 10 bis 20 Kohlenstoffatomen. Der Alkoxyrest kann geradkettig oder verzweigt, gesättigt oder teilweise ungesättigt sein.

Halogen in der Definition der Reste R²⁰, R²¹, R²² kann Fluor, Chlor, Brom oder Jod bedeuten, bevorzugt sind Chlor und Brom.

Eine Alkylsulfonylgruppe in der Definition der Reste R¹⁹, R²⁰, R²¹, R²² bedeutet die Gruppierung Alkyl-SO₂-. In diesem Zusammenhang stellt die Alkylgruppe einen Alkylrest aus 1 bis 4, vorzugsweise 1 bis 2 Kohlenstoffatomen dar. Besonders bevorzugt ist die Methylsulfonylgruppe.

Unter einer Sulfonamidgruppe in der Definition des Restes R¹⁹ wird ein unsubstituiertes Amid (-SO₂NH₂) oder ein Amid eines primären oder sekundären Amins (-SO₂NHR oder -SO₂NR₂) verstanden. Substituenten des Amids (R) können Alkyl, Aryl oder Aralkylreste sein.

Im Falle des Amids eines sekundären Amins können die Substituenten (R) gleich oder verschieden sein. Unter einem Alkylrest wird in diesem Zusammenhang ein Rest mit 1 bis 4 Kohlenstoffatomen verstanden. Ein Arylrest bedeutet einen aromatischen Rest mit 6 bis 10 Kohlenstoffatomen. Bevorzugte Arylreste sind der Phenyl- oder Naphthylrest. Aralkylreste sind solche Reste, in denen der Arylteil ein aromatischer Rest mit 6 bis 10 Kohlenstoffatomen und der Alkylteil ein Rest mit 1 bis 4 Kohlenstoffatomen ist. Bevorzugter Aralkylrest ist der Benzylrest. Ganz besonders bevorzugt ist die unsubstituierte Sulfonamidgruppe (-SO₂NH₂).

Besonders bevorzugte Naphtholderivate der allgemeinen Formel IV sind solche, bei denen einer der Reste R¹⁵, R¹⁶ und R¹⁷ einen Alkyl- oder Alkoxyrest mit 8 bis 30, vorzugsweise 10 bis 20 Kohlenstoffatomen darstellt und die übrigen Reste aus der vorgenannten Gruppe Wasserstoff oder einen Alkylrest mit 1 bis 4, vorzugsweise 1 bis 2 Kohlenstoffatomen bedeuten.

Ganz besonders bevorzugte Naphtholderivate sind solche, in denen R¹⁵ eine Alkoxygruppe mit 10 bis 20 Kohlenstoffatomen, R¹⁶ und R¹⁷ Wasserstoff darstellen und die übrigen Reste die für Formel IV angegebene Bedeutung haben.

Die Verbindungen der allgemeinen Formel IV können nach an sich bekannten Verfahren analog hergestellt werden. Zur Herstellung von Naphtholderivaten der allgemeinen Formel IV sind mehrere Verfahrensvarianten möglich. Zum einen können Naphthochinone der allgemeinen Formel V 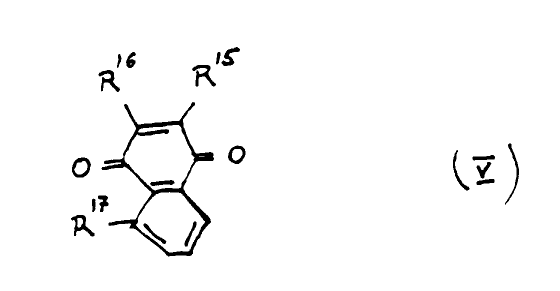 in der
R¹⁵, R¹⁶ und R¹⁷ die für die allgemeine Formel IV angegebene Bedeutung haben, mit einem Hydrazin der allgemeinen Formel VI 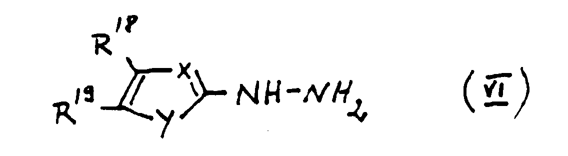 in der R¹⁸, R¹⁹, X und Y die für die allgemeine Formel IV angegebene Bedeutung haben umgesetzt werden. Diese Umsetzung kann unter den für die Bildung eines Hydrazons üblichen Bedingungen erfolgen. Vorzugsweise erfolgt die Reaktion unter sauren Bedingungen. Das Hydrazon selbst ist nicht beständig, sondern lagert sich zu dem gewünschten Naphthol der allgemeinen Formel IV um.

Ein anderer Weg zur Herstellung der erfindungsgemäßen Naphtholderivate der allgemeinen Formel IV geht aus von Aminen der allgemeinen Formel VII 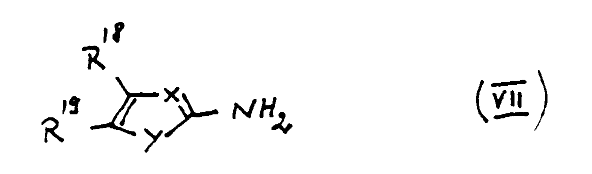 in der R¹⁸, R¹⁹, X und Y die für die Formel IV angegebene Bedeutung haben. Diese Amine werden diazotiert und die resultierenden Diazoniumsalze mit einem Naphthol der allgemeinen Formel VIII 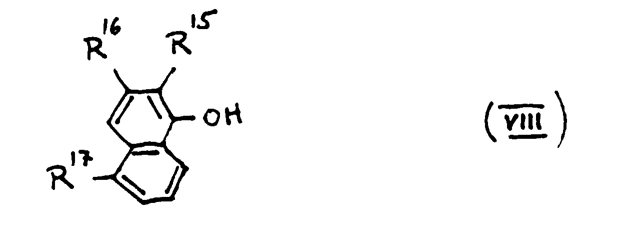 in der R¹⁵, R¹⁶ und R¹⁷ die für die allgemeine Formel IV angegebene Bedeutung haben, in einer Azokupplungsreaktion umgesetzt.

Die Diazotierung der Amine der allgemeinen Formel VII kann in der üblichen Weise erfolgen. Als vorteilhaft erwiesen hat es sich, konzentrierte Mineralsäure, beispielsweise konzentrierte Schwefelsäure mit einem Nitrit, vorzugsweise Natriumnitrit vorzulegen und unter Kühlung auf Raumtemperatur das Amin der allgemeinen Formel VII zuzugeben. Als ganz besonders vorteilhaft hat sich eine Diazotierungsmischung erwiesen, die Natriumnitrit und neben konzentrierter Schwefelsäure auch Eisessig enthält. Das bevorzugte Volumenverhältnis von Schwefelsäure und Eisessig liegt zwischen 1:1 und 2:1. Üblicherweise ist das Verhältnis von Nitrit und zu diazotierendem Amin der allgemeinen Formel VII äquimolar.

Nach beendeter Diazotierungsreaktion wird die Reaktionsmischung wässrig aufgearbeitet. Vorteilhafterweise gießt man hierzu die Reaktionsmischung auf Eiswasser. Das Diazoniumsalz selbst wird nicht isoliert, sondern in der wässrigen Aufarbeitungslösung mit dem Naphthol der allgemeinen Formel VIII zur Azokupplung gebracht. Diese erfolgt vorzugsweise unter schwach sauren Bedingungen. Naphthole der allgemeinen Formel VIII sind in wässrigen Lösungen nur sehr schwer löslich. Sie werden deshalb in organischen Lösungsmitteln zum Einsatz gebracht. Als organisches Lösungsmittel ist beispielsweise Chloroform gut geeignet. So kann eine nach wässriger Aufarbeitung vorliegende Diazoniumsalzlösung zu einer Lösung eines Naphthols der allgemeinen Formel VIII in Chloroform und Eisessig und Zugabe eines Acetats zur Abpufferung des pH-Wertes des Reaktionsmediums hinzugefügt werden. Meist fallen die gebildeten Naphtholderivate der allgemeinen Formel IV aus der Reaktionsmischung aus. Das Produkt kann dann umkristallisiert oder chromatographisch aufgereinigt werden.

Die pH-Indikatoren der allgemeinen Formel IV sind in wässrigen Lösungen nur schlecht, umso besser aber in organischen Lösungsmitteln löslich.

Erfindungsgemäß hat sich die Kombination von 4-(2,6-Dibrom-4-nitro-phenylazo)-2-octadecyloxy-naphthol-1 als pH-Indikator mit 2,4,6,8-Tetranitro-5-octadecyloxy-naphthol-1 als Säure als hervorragend geeignet erwiesen.

In Kombination mit einem pH-Indikator eignen sich die erfindungsgemäßen Säuren der allgemeinen Formeln I, II und III sehr gut zur Erhöhung der Meßempfindlichkeit von Verfahren zur Bestimmung eines Ions in einer wässrigen Flüssigkeit nach dem Prinzip der "heterogenen pH-Reaktion" wie es eingangs beschrieben worden ist. Sie können hierzu sowohl in Flüssig-Flüssig-Extraktionen, wie sie grundsätzlich bei E.S. Hyman, Biophysical Society Abstracts, 1971, 72a beschrieben sind, eingesetzt werden, als auch auf "trockenchemischen" Testträgern, die nach dem Prinzip der heterogenen pH-Reaktion arbeiten. Verfahren zur Bestimmung eines Ions in einer wässrigen Flüssigkeit, die nach dem Prinzip der heterogenen pH-Reaktion ablaufen, ist es gemeinsam, daß das zu bestimmende Ion aus der wässrigen Probenflüssigkeit in eine mit der wässrigen Flüssigkeit nicht mischbare Phase gelangt und ein dort anwesender pH-Indikator dadurch eine Farbänderung erfährt, welche zur Bestimmung des Ions herangezogen wird.

Ein erfindungsgemäßes Mittel zur Bestimmung eines Ions in einer wässrigen Flüssigkeit enthält in einem organischen Medium, das mit Wasser nicht mischbar ist, neben einem in organischem Medium löslichen pH-Indikator einen für den Transport des zu bestimmenden Ions aus der wässrigen Flüssigkeit in die organische Phase verantwortlichen Ionophor und eine erfindungsgemäße Säure der allgemeinen Formeln I, II oder III. Im folgenden wird in diesem Zusammenhang näher auf "trockenchemische" Mittel zur Bestimmung von Ionen eingegangen. Für den Fachmann ist es jedoch selbstverständlich, daß im Grunde die folgenden Ausführungen analog auch für Flüssig-Flüssig-Extraktionen und somit nicht-testträgergebundenen Testverfahren gelten.

Testträger zur Bestimmung von Ionen, die auf dem Prinzip der heterogenen pH-Reaktion basieren, sind wie einleitend beschrieben, aus dem Stand der Technik bekannt. Sie unterscheiden sich im wesentlichen in ihrer Ausführungsform, d. h. wie die organische Phase in einer für Testträger sinnvollen Form realisiert ist. Generell besteht die organische Phase aus schwer flüchtigen, mit Wasser nicht mischbaren organischen Flüssigkeiten und/oder hydrophoben Polymeren. Liegen beide zusammen als feste Lösung vor, so spricht man auch von weichgemachten Kunststoffen.

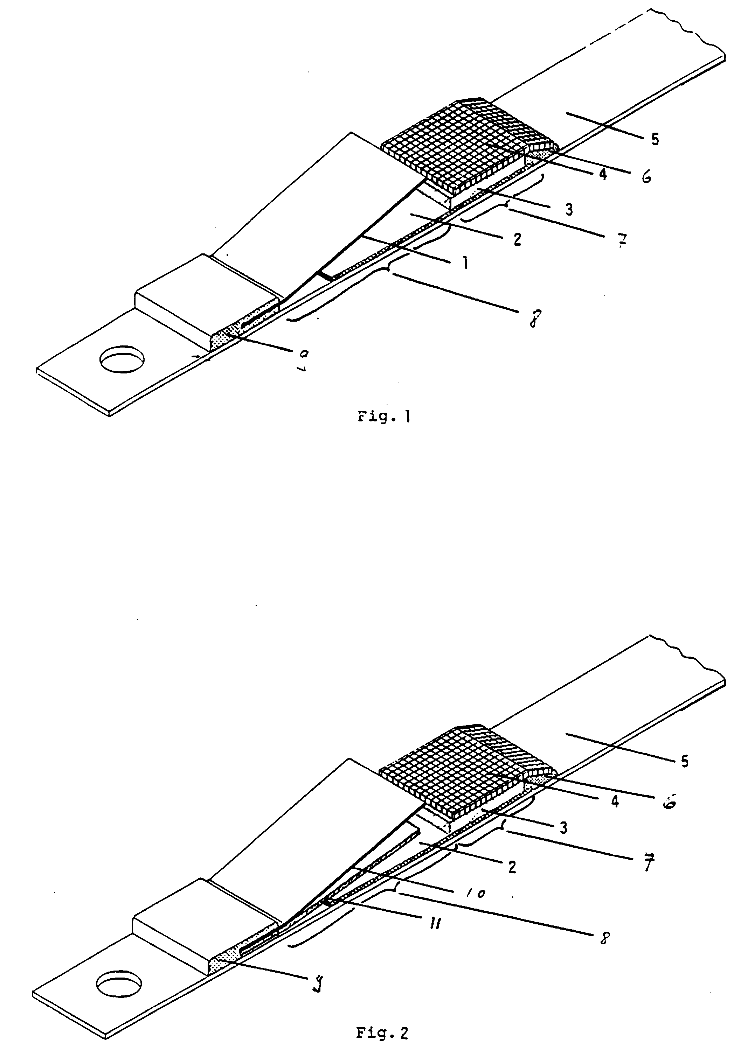

Die erfindungsgemäßen Säuren der allgemeinen Formeln I, II oder III können prinzipiell in allen aus dem Stand der Technik bekannten Testträgern zur Bestimmung von Ionen, mit denen das Prinzip der heterogenen pH-Reaktion durchführbar ist, eingesetzt werden. Ganz besonders vorteilhaft haben sie sich jedoch in Testträgern erwiesen, bei denen sie zusammen mit einem Naphtholderivat der allgemeinen Formel IV in einer Filmschicht vorliegen, die einen flüssigkeitsfesten Film aus einem hydrophoben Polymer und darin verteilte, feste inerte Partikel enthält. Solche Testträger sind in DE-A-4015590.0 beschrieben. Beispiele für solche Testträger sind in den Fig. 1 und 2 dargestellt.

In den Figuren 1 und 2 sind zwei Testträger räumlich dargestellt, die für die Bestimmung von Ionen in Blut geeignet sind. Sie erlauben, aus Vollblut Serum bzw. Plasma abzutrennen und in der so gewonnenen Flüssigkeit die Bestimmung der interessierenden Ionen durchzuführen. Die Testträger unterscheiden sich im wesentlichen nur durch die Anordnung der Puffersubstanz innerhalb des Testträgers. Im einzelnen sind die Vorrichtungen wie folgt zusammengesetzt:

Fig. 1: Auf einer inerten Trägerfolie (5), beispielsweise einer Kunststoffolie, ist eine Transportschicht (2) befestigt, die zum Transport der Probenflüssigkeit aus der Probenaufgabezone (7) in den Nachweisbereich (8) dient. Als Transportschicht (2) ist prinzipiell jedes Material geeignet, das die zu untersuchende Flüssigkeit aus der Probenaufgabezone (7) in den Nachweisbereich (8) zu transportieren in der Lage ist und sie dabei nicht so verändert, daß die Analyse beeinträchtigt wird. Besonders vorteilhaft ist es, als Transportschicht (2) ein Glasfaservlies einzusetzen. Die Transportschicht (2) teilweise überdeckend ist eine Schicht (3) zur Abtrennung von korpuskulären Bestandteilen aus der Probenflüssigkeit befestigt. Grundsätzlich kann hierfür jedes Material verwendet werden, das erlaubt, korpuskuläre Bestandteile aus der Probenflüssigkeit, insbesondere Blutzellen, vor allem Erythrozyten aus Blut, abzutrennen und diese nicht in wesentlichen Mengen in den Nachweisbereich (8) gelangen zu lassen, so daß sie dort keine Störung der Nachweisreaktion verursachen. Im übrigen darf die Abtrennschicht (3) nicht zu einer Veränderung der Probenflüssigkeit dahingehend führen, daß darin die Konzentration der zu bestimmenden Ionen verändert und so das Ergebnis verfälscht wird. Als besonders geeignet für die Abtrennschicht (3) haben sich Glasfaservliese erwiesen, wie sie z. B. in EP-B-0 045 476 beschrieben sind. Über der Abtrennschicht (3) ist eine Schutzschicht (4) angebracht, die verhindern soll, daß bei der Probenaufgabe, beispielsweise mittels einer Pipette, die Abtrennschicht (3) beschädigt wird. Bewährt hat sich hierfür ein Netz aus inertem Material, beispielsweise aus Kunststoff. Schutzschicht (4) und Abtrennschicht (3) sind auf der inerten Trägerfolie (5) befestigt. Dies kann beispielsweise mittels eines Schmelzklebestreifens (6) erfolgen. Seitlich von der Transportschicht (2) ist eine aus durchsichtigem Kunststoff bestehende Trägerfolie mit einer Filmschicht (1), die die zur Durchführung der Bestimmung notwendigen Reagenzien (auch eine erfindungsgemäße Verbindung der allgemeinen Formeln I, II oder III) enthält, befestigt. Vorteilhafterweise geschieht dies über eine Klebestelle (9), beispielsweise einen Schmelzkleberstreifen. Die Filmschicht (1) ist so angeordnet, daß sie beim Niederdrücken der durchsichtigen Trägerfolie in Richtung der inerten Trägerfolie (5) mit der Transportschicht (2) in einen einen Flüssigkeitsübergang ermöglichenden Kontakt gebracht werden kann.

Die Filmschicht (1) enthält einen flüssigkeitsfesten Film aus einem hydrophoben Polymer und darin verteilten Partikeln. Das hydrophobe Polymer ist für die zu untersuchende Flüssigkeit und auch für zu bestimmende Ionen nicht durchlässig. Es sind die Partikel, die ein Eindringen der Probenflüssigkeit in die Filmschicht ermöglichen. Die Filmschicht (1) insgesamt ist für die zu untersuchende Flüssigkeit nicht durchlässig. Es wird lediglich ein bestimmtes Volumen aufgenommen. Als vorteilhaft einsetzbare hydrophobe Polymere haben sich insbesondere Copolymere des Vinylacetates erwiesen. Besonders vorteilhaft sind Copolymere des Vinylacetats mit Vinyllaurat oder Maleinsäuredibutylester.

Als Partikel können feste, in den zu untersuchenden Flüssigkeiten unlösliche, inerte, anorganische oder organische Partikel eingesetzt werden, die eine Ölzahl von 80 - 200, vorzugsweise 100 - 170 aufweisen. Vor allem haben sich die verschiedenen Arten von Diatomeenerde, wie ungeglühte oder Naturkieselgur, calcinierte oder gebrannte Kieselgur, flußgeglühte oder aktivierte Kieselgur als vorteilhaft für die Filmschicht (1) erwiesen.

Die Ölzahl ist eine aus dem Gebiet der Lacke und Beschichtungen bekannte Kenngröße für Teilchen, die beispielsweise als Füllstoffe eingesetzt werden. Sie ist ein Maß für die Wechselwirkung zwischen den Teilchen und dem Medium in dem sie dispergiert werden. Die Ölzahl ist einfach zu ermitteln. Die Bestimmung erfolgt nach DIN 53 199. Nach dieser Norm gibt die Ölzahl die Menge Leinöl in g an, die gebraucht wird, um 100 g der interessierenden Partikel zu einer zusammenhängenden kittartigen Masse zu verarbeiten.

Die eingesetzten Teilchen haben in der Regel eine ungleichmäßige Gestalt. Ihre Korngröße liegt üblicherweise zwischen 0,1 und 200 µm, vorzugsweise zwischen 0,2 und 30 µm. Insbesondere zeichnen sich die erfindungsgemäßen Partikel dadurch aus, daß sie Hohlräume aufweisen, in die Gase und benetzende Flüssigkeiten eindringen können. Diese Eigenschaft drückt sich insbesondere in dem niedrigen Schüttgewicht von 50 bis 250, vorzugsweise 80 bis 180 g/l aus.

Für die Filmschicht (1) ist ein Gewichtsverhältnis von hydrophobem Polymer zu Partikel von 5:1 bis 1:10 brauchbar. Vorteilhafterweise beträgt das Gewichtsverhältnis 1:1 bis 1:3. In jedem Fall ist das optimale Gewichtsverhältnis von hydrophobem Polymer zu Partikel von der Natur des eingesetzten Polymers und der Teilchen abhängig. Ist das hydrophobe Polymer ein Copolymer des Vinylacetats mit Vinyllaurat und/oder Maleinsäuredibutylester und die Teilchen Diatomeenerde liegt das optimale Gewichtsverhältnis zwischen 1:1,5 und 1:2,5.

Weitere notwendige Bestandteile der Filmschicht (1) sind eine schwer flüchtige mit Wasser nicht mischbare Flüssigkeit, ein Ionophor, ein pH-Indikator und zur Erhöhung der Meßempfindlichkeit eine Säure der allgemeinen Formeln I, II oder III. Diese Komponenten liegen in homogener Verteilung in dem hydrophoben Polymer vor.

Unter einer schwer flüchtigen, mit Wasser nicht mischbaren Flüssigkeit wird ein Weichmacher für Kunststoffe verstanden. Er dient zusammen mit dem Polymer als eigentliche organische Phase für das Ionenbestimmungsverfahren gemäß dem Prinzip der heterogenen pH-Reaktion. Es kommen alle möglichen handelsüblichen Typen von Kunststoffweichmachern, vorzugsweise Sebacinsäure-, Acrylsäure-, Phthalsäure- und Phosphorsäure-Ester sowie Silicone in Frage. Insbesondere aus verarbeitungstechnischen Gründen wird dem sehr schwer flüchtigen Uvinul®N539 (2,2-Diphenyl-1-cyanoacrylsäure-ethylhexylester) der Vorzug gegeben.

Das Gewichtsverhältnis von hydrophobem Polymer zu schwer flüchtiger, hydrophober organischer Flüssigkeit in der Testschicht kann zwischen etwa 5:1 bis etwa 1:5, insbesondere etwa 2:1 bis etwa 1:2 betragen.

Als Ionophore sind alle die Ionen komplexierenden Substanzen verwendbar, die für die zu bestimmenden Ionen spezifisch und hinreichend löslich in nicht-wässriger Phase sind. Hierbei ist insbesondere an Kronenäther, Kryptanden, Podanden und entsprechende Peptide cyclischer oder acylischer Natur zu denken. Für die Bestimmung von Kalium hat sich 2,3-Naphtho-15-Krone-5 als vorteilhaft erwiesen. Ganz besonders bevorzugt ist das Naturstoffionophor Valinomycin. Für die Bestimmung von Natrium kommen beispielsweise N,N'-Dibenzyl-N,N'-diphenyl-1,2-phenylendioxydiacetamid, für Lithium N,N'-Diheptyl-5,5-dimethyl-N,N'-di(3-oxapentyl)-3,7-dioxanonandiamid und für Calcium Diethyl-N,N'-[(4R,5R)-4,5-dimethyl-1,8-dioxo-3,6-dioxa-octamethylen]-bis-(12-methylaminododecanoat) in Frage. Naturgemäß sind die Ionophore nicht zu verwenden, die basische Stickstoffatome enthalten, die durch die erfindungsgemäßen Säuren protoniert werden und dadurch ihre selektive Komplexbildungsfähigkeit für das zu bestimmende Ion verlieren. Dies trifft insbesondere für Kryptanden zu. Die weitaus meisten Ionophore enthalten jedoch keinen unter den Testbedingungen protonierbaren Stickstoff, so daß die Universalität der Erfindung kaum eingeschränkt wird.

Als pH-Indikatoren kommen grundsätzlich alle infrage, die hinreichend löslich in der organischen Phase und so hydrophob sind, daß sie mit der zu untersuchenden wässrigen Probe nicht aus der organischen Phase heraus extrahiert werden. Beispielsweise können Tetrabromphenolphthaleinester oder die in EP-A-0 128 317 und EP-A-0 128 318 beschriebenen Indonaphtholderivate mit verschieden langen Alkyl-Seitenketten eingesetzt werden. Auch Chromoionophore sind einsetzbar. Besonders gut geeignet sind jedoch Naphtholderivate der allgemeinen Formel IV, wie sie vorstehend bereits charakterisiert wurden.

Aufgrund des Einsatzes von pH-Indikatoren und deren Empfindlichkeit gegen pH-Änderungen ist es besonders vorteilhaft, in die Filmschicht (1) auch einen Puffer einzubringen. Der pH-Wert eines Puffers regelt bei Bestimmungsverfahren für Ionen, die auf dem Prinzip der heterogenen pH-Reaktion beruhen, den übertritt des Protons aus der nicht-wässrigen in die wässrige Phase. Vorzugsweise wählt man bei diagnostischen Mitteln für die Bestimmung von Ionen in Körperflüssigkeiten die Puffersubstanz so, daß ein pH-Wert zwischen 5 - 10, vorzugsweise 7 bis 8, eingestellt werden kann. Hierfür kommen prinzipiell alle hierfür üblichen Puffer in Frage, sofern sie wasserlöslich sind und keine Ionen enthalten, die die Bestimmungsreaktion stören. Bewährt haben sich Puffer aus der Reihe der sogenannten Good-Puffer, wie z. B. N,N-Bis-(hydroxyethyl)-aminoethansulfonsäure (BES), 3-[N-Trishydroxymethyl]-methylamino-hydroxypropansulfonsäure (TAPSO), oder N-Hydroxyethylpiperazin-N-propansulfonsäure (HEPPS).

Werden Ionophore verwendet, die nicht genügend selektiv für das zu bestimmende Ion sind, so können wasserlösliche Komplexbildner zugesetzt werden, die störende Ionen maskieren. So wird z. B. eine etwaige Störung eines Natriumtestes durch Calcium mit Ethylendiamintetraacetat (EDTA) verhindert.

Weiter können zur Verbesserung der Filmherstellung oder der Filmbenetzung durch die zu untersuchende Probe Netzmittel eingesetzt werden. Hierzu sind nur solche verwendbar, die die Nachweisreaktion nicht stören. Dies sind nichtionische und zwitterionische Verbindungen. Von den nichtionischen Netzmitteln haben sich beispielsweise Polyethylenglykolether oder -ester, vorzugsweise Triton® X100 als vorteilhaft erwiesen. Als zwitterionisches Netzmittel kann vorteilhafterweise n-Decyl-N,N-dimethyl-3-ammonio-1-propansulfonat (Zwittergent®3-10) eingesetzt werden.

Zur Verbesserung der Konsistenz der Filmschicht (1) können zusätzlich auch Verdicker eingesetzt werden. Als besonders vorteilhaft hat sich hier Ethylcellulose erwiesen. Für die nach der Benetzung der Filmschicht (1) mit einer zu analysierenden wässrigen Flüssigkeit vorliegende wässrige Phase kann der Filmschicht (1) auch noch zusätzlich hydrophiles Verdickungsmittel, wie beispielsweise Hydroxyethyl- oder Hydroxypropyl-cellulose zugegeben werden.

Zur Herstellung einer Filmschicht (1) werden alle Komponenten, die bei einem Einsatz der Filmschicht zur Bestimmung eines Ions in einer wässrigen Flüssigkeit, insbesondere einer Körperflüssigkeit, wie Blut, Plasma, Serum oder Urin, nicht in die wässrige Phase aufgenommen werden, sondern in der organischen Phase, d. h. der Filmschicht bleiben (hydrophobes Polymer; schwer flüchtige, mit Wasser nicht mischbare Flüssigkeit; Ionophor; pH-Indikator; erfindungsgemäße Säure; gegebenenfalls Verdicker zur Verbesserung der Konsistenz der Filmschicht) in einem leicht- bis mittelflüchtigen organischen Lösungsmittel gelöst. In diese Lösung werden die Partikel eingerührt und darin homogen verteilt. Die Masse wird danach auf einer Unterlage mit einem Rakel ausgestrichen und getrocknet. Es können naturgemäß auch andere geeignete Auftragsverfahren, wie Walzenauftrag, Filmgießen oder ähnliche angewendet werden. Die trockene Filmschicht hat eine Dicke von 20 bis 500, vorzugsweise von 20 bis 150 µm.

Zur Einbringung von Bestandteilen, die bei Aufgabe der wässrigen Probenflüssigkeit auf die Filmschicht (1) in die wässrige Phase aufgenommen werden (Puffer; gegebenenfalls Komplexbildner; gegebenenfalls Netzmittel; gegebenenfalls Verdicker zur Veränderung der Konsistenz der wässrigen Phase) gibt es verschiedene Wege. Eine Möglichkeit ist es, die Partikel mit den genannten Komponenten zu belegen, indem man die Teilchen zusammen mit einer wässrigen Lösung der Komponenten eindampft, sprühtrocknet oder gefriertrocknet. Die so belegten Partikel werden dann wie oben beschrieben in die organische Lösung eingerührt. Eine andere Möglichkeit besteht darin, die Filmschicht erst mit unbehandelten Partikeln herzustellen, dann mit einer wässrigen Lösung der vorstehend genannten Bestandteile nachzubeschichten und abschließend zu trocknen.

Fig. 2 unterscheidet sich von Fig. 1 darin, daß über die Klebestelle (9), beispielsweise einen Schmelzkleberstreifen, zwischen Filmschicht (10) und Transportschicht (2) eine Schicht (11) angebracht ist, die solche Substanzen enthält, die während der Bestimmungsreaktion in die wässrige Phase aufgenommen werden. Solche Substanzen sind insbesondere Puffersubstanzen. Aber auch Komplexbildner, Netzmittel oder Verdicker zur Veränderung der Konsistenz der wässrigen Phase können statt in der Filmschicht (1) des Testträgers gemäß Fig. 1 oder (10) des Testträgers gemäß Fig. 2 in der zusätzlichen Schicht (11) des Testträgers gemäß Fig. 2 untergebracht sein. Als Material für die zusätzliche Schicht (11) kommen saugfähige Materialien in Frage, die einen Flüssigkeitsübergang auf eine weitere Schicht ermöglichen, wenn diese in Kontakt mit ihr gebracht wird. Besonders vorteilhaft ist Papier einsetzbar, aber auch Netze aus inertem Material, wie Kunststoff sind möglich.

Zur Durchführung der Bestimmung eines Ions in Blut mittels eines der in den Figuren dargestellten Testträgers wird die Probe auf die Schutzschicht (4) gegeben. Das Blut dringt in die Abtrennschicht (3) ein und Erythrozyten werden von Plasma bzw. Serum getrennt. Durch Kapillarkräfte wird die so gewonnene Flüssigkeit in die Nachweiszone (8) gesaugt. Durch Druck auf die Trägerfolie mit der Filmschicht (1) bzw. (10) wird die wässrige Phase in der Transportschicht (2) mit der Filmschicht in Kontakt gebracht, Flüssigkeit dringt in die Filmschicht ein und die Bestimmungsreaktion wird ausgelöst. Die Reaktion wird anhand der Verfärbung in der Filmschicht durch die Trägerfolie der Filmschicht (1) bzw. (10) hindurch visuell beobachtet oder reflexionsphotometrisch gemessen.

Die folgende Tabelle 1 gibt die vorteilhaften und bevorzugten Gewichtsprozente der Bestandteile einer Filmschicht (1) bzw. (10) an:

**Tabelle 1:**

| Bestandteil der Filmschicht | Gehalt der Filmschicht in Gew.-% | |
|---|---|---|
| | vorteilhaft | bevorzugt |
| Polymer | 5 - 60 | 20 - 40 |
| | | |
| schwerflüchtige, mit Wasser nicht mischbare Flüssigkeit | 5 - 70 | 20 - 40 |
| | | |
| Partikel | 15 - 80 | 30 - 50 |
| | | |
| Ionophor | 0,05 - 5,0 | 0,2 - 1,0 |
| | | |
| pH-Indikator | 0,05 - 5,0 | 0,2 - 0,7 |
| | | |
| erfindungsgemäße Säure | 0,005 - 5,0 | 0,02 - 0,7 |

Ist Puffersubstanz in oder auf die Filmschicht (1) gebracht, so enthält diese 5 - 30, vorzugsweise 10 - 20 Gew.-% Puffer. Die gegebenenfalls einsetzbaren Substanzen, wie Komplexbildner, Netzmittel oder Verdicker, liegen - falls sie in die Filmschicht (1) ein- oder aufgebracht worden sind - in Mengen von 0,005 bis 5, vorzugsweise 0,02 bis 2 Gew.-% der erfindungsgemäßen Filmschicht vor.

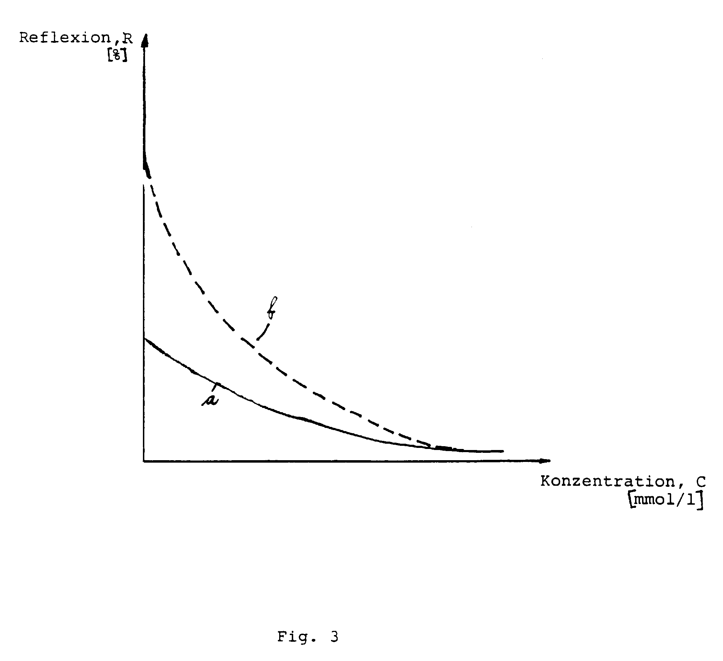

Auf Testträgern kann die Farbänderung des pH-Indikators als Maß für die Menge des zu bestimmenden Ions visuell ausgewertet werden. Genauer ist eine Auswertung jedoch durch reflexionsphotometrische Messung möglich. In Fig. 3 ist verallgemeinernd eine typische Kurve (a) angegeben, die den Zusammenhang zwischen Reflexion (R) und Konzentration (c) widergibt, wie er mit einem Testträger zur Bestimmung eines Ions in einer wässrigen Flüssigkeit ermittelt wird. Während Kurve a den Verlauf widergibt, der sich ohne Zusatz einer erfindungsgemäßen Säure ergibt, stellt Kurve b den Zusammenhang zwischen Reflexion und Konzentration dar, wenn neben einem pH-Indikator noch zusätzlich eine erfindungsgemäße Säure in der organischen Phase anwesend ist. Kurve b (mit Säure) ermöglicht genauere Konzentrationsermittlungen, da die Steigung der Kurve b größer ist als die von a. Aus Fig. 3 (Kurve b) ist somit ersichtlich, daß durch Kombination einer Säure der allgemeinen Formeln I, II oder III mit einem pH-Indikator in einer Ionenbestimmung die auf dem Prinzip der heterogenen pH-Reaktion beruht, eine Empfindlichkeitssteigerung gegenüber solchen Testen ohne Säure möglich ist.

Verbindungen der allgemeinen Formel I' 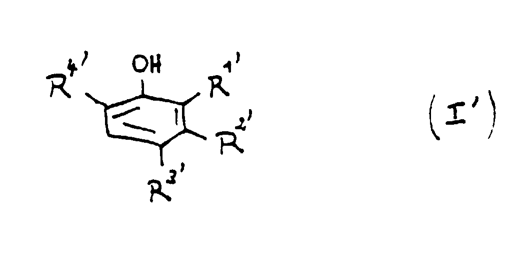 in der
- R^{2'}: einen Alkyl- oder Alkoxyrest darstellt und
- R^{1'}, R^{3'} und R^{4'}: gleich oder verschieden sind und jeweils eine Nitrogruppe, Halogen, eine Cyanogruppe, eine Alkylsulfonylgruppe oder eine mit Halogen substituierte Alkylgruppe bedeuten,
sollen ebenfalls Teil der vorliegenden Erfindung sein.

Die Bedeutungen der Definitionen der Reste R^{1'} bis R^{4'} entsprechen den für die Reste R¹ bis R⁴ der allgemeinen Formel I gegebenen.

Die Verbindungen können analog zu bekannten Verfahren hergestellt werden. Insbesondere sind sie durch aromatische Substitution von entsprechenden Ausgangsverbindungen zugänglich. Verbindungen der allgemeinen Formel I', in denen R^{1'}, R^{3'} und R^{4'} alle gleich sind und jeweils eine Nitrogruppe bedeuten, können beispielsweise so hergestellt werden, daß eine Verbindung der allgemeinen Formel IX 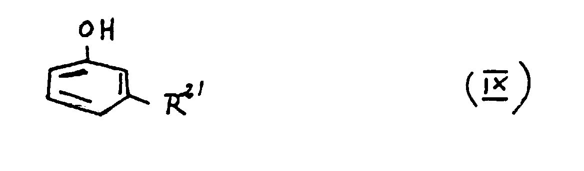 in der R²' einen Alkyl- oder Alkoxyrest darstellt, zuerst mit Schwefelsäure und dann mit Salpetersäure versetzt wird. Insbesondere mit konzentrierter Schwefelsäure findet zunächst eine Sulfonierung der Ausgangssubstanz statt. Gegebenenfalls muß hierzu bis auf 90 °C erwärmt werden. Bei der nachfolgenden Nitrierung, insbesondere bei Temperaturen unterhalb bis maximal Raumtemperatur, vorzugsweise 0 bis 25 °C, findet Einführung der Nitrogruppen in die gewünschten Positionen statt.

Die Verbindungen der allgemeinen Formel II,
in der
- R⁷: einen Alkyl-, Alkoxy- oder Aralkylrest, insbesondere einen
Alkoxyrest darstellt und die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können, sind ebenfalls neu. Die Bedeutungen der Definitionen für die Reste R⁵ bis R⁹ entsprechen den eingangs gegebenen.

Die Verbindungen der allgemeinen Formel II sind analog zu bekannten Verfahren herstellbar. Insbesondere sind sie (analog zu Verbindungen der allgemeinen Formel I') durch aromatische Substitution von entsprechenden Ausgangsverbindungen zugänglich.

Verbindungen der allgemeinen Formel II, in denen die Reste R⁵, R⁶, R⁸ und R⁹ gleich sind und jeweils eine Nitrogruppe bedeuten, können beispielsweise so hergestellt werden, daß eine Verbindung der allgemeinen Formel X 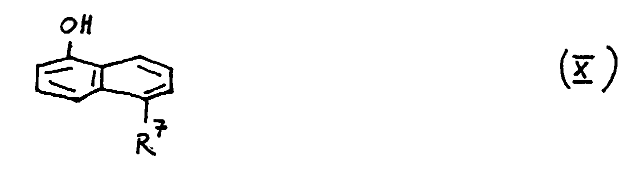 in der R⁷ einen Alkyl-, Alkoxy- oder Aralkylrest, insbesondere einen Alkoxyrest darstellt, zuerst mit Schwefelsäure und dann mit Salpetersäure versetzt wird. Insbesondere mit konzentrierter Schwefelsäure findet zunächst eine Sulfonierung der Ausgangsverbindung statt. Gegebenenfalls muß hierzu bis auf 50 °C erwärmt werden. Bei der nachfolgenden Nitrierung, insbesondere bei Temperaturen unterhalb bis maximal Raumtemperatur, vorzugsweise 0 - 10 °C, findet Ersatz der Sulfonsäuregruppen und Einführung der Nitrogruppen statt.

Wird die Verbindung der allgemeinen Formel X direkt mit Salpetersäure, vorzugsweise in Eisessig bei Raumtemperaturen, umgesetzt, erhält man eine Verbindung der allgemeinen Formel XI 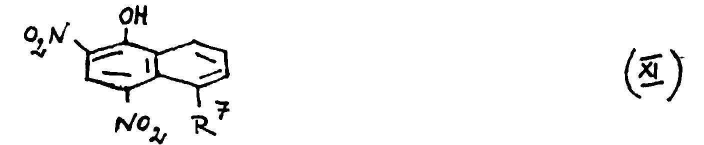 in der R⁷ die gleiche Bedeutung wie für Verbindungen der allgemeinen Formel X hat.

Neue Verbindungen aus der Gruppe der Verbindungen der allgemeinen Formel III sind
[(2,3,5,6-Tetrafluorphenyl)-hydrazono]propandinitril,
[(2-Trifluormethyl-4-nitrophenyl)-hydrazono]propandinitril,
[(2-Methansulfonyl-4-nitrophenyl)-hydrazono]propandinitril,
[(2,4-Dinitro-6-cyanophenyl)-hydrazono]propandinitril und
[(3,5-Di-{trifluormethyl}phenyl)-hydrazono]propandinitril.

Sie sind ebenfalls Gegenstand der vorliegenden Erfindung.

Sie können hergestellt werden, indem ein entsprechendes Anilinderivat (2,3,5,6-Tetrafluoranilin, 2-Trifluormethyl-4-nitroanilin, 2-Methansulfonyl-4-nitroanilin, 2,4-Dinitro-6-cyanoanilin, 3,5-Di-(trifluormethyl)-anilin) in das entsprechende Diazoniumsalz überführt wird und mit Malondinitril umgesetzt wird.

Die Diazotierung der Anilinderivate kann in üblicher Weise erfolgen. Als vorteilhaft erwiesen hat es sich, konzentrierte Mineralsäure, beispielsweise konzentrierte Schwefelsäure mit einem Nitrit, vorzugsweise Natriumnitrit, vorzulegen und unter Kühlung auf Raumtemperatur das Anilinderivat zuzugeben. Als ganz besonders vorteilhaft hat sich eine Diazotierungsmischung erwiesen, die Natriumnitrit und neben konzentrierter Schwefelsäure auch Eisessig enthält. Das bevorzugte Volumenverhältnis von Schwefelsäure und Eisessig liegt zwischen 1:1 und 2:1. Üblicherweise ist das Verhältnis von Nitrit und zu diazotierendem Anilin äquimolar.

Die so hergestellte Diazoniumsalzlösung wird zu einer wässrigen, vorzugsweise acetatgepufferten Lösung von Malondinitril zugegeben. Die gebildeten Propandinitrilderivate fallen in der Regel aus der Reaktionsmischung aus und können dann durch Umkristallisation oder chromatographische Verfahren aufgereinigt werden.

In den folgenden Beispielen wird die Erfindung weiter erläutert:

### Beispiel 1

### 3-Pentadecyl-2 4 6-trinitrophenol

In einem 500 ml Erlenmeyerkolben werden 67,5 g (0,2 mol) Pentadecylphenol 90 % unter Rühren in 100 ml konz. Schwefelsäure eingetragen, auf 90°C erwärmt, wobei eine schwarzbraune, hochviskose, schwer rührbare Masse entsteht, die eine Stunde bei 90°C gehalten wird. In einem separaten 1 l Dreihalskolben werden 70 ml (ca. 1 mol) 65proz. Salpetersäure mit Hilfe eines Eisbades auf 10°C gekühlt. Das hochviskose zuvor erhaltene Sulfonierungsprodukt wird während ca. 2 Stunden in kleinen Portionen (wobei die viskose Masse mit einem Fön flüssig gehalten wird) unter Eisbadkühlung so zu der Salpetersäure gegeben, daß die Temperatur von 25°C nicht überschritten wird und erhält dabei eine beigefarbene schwer rührbare Masse, die man eine Stunde bei Raumtemperatur weiterrührt. Danach gießt man auf 500 g Eis, wobei sich ein feiner Niederschlag bildet. Das so erhaltene Rohprodukt läßt sich nur schwer absaugen, weshalb man das Ganze am besten an der Zentrifuge schleudert. Nach Abdekantieren der überstehenden Flüssigkeit wird diese Prozedur zur Entfernung von anhaftender Säure zweimal nach jeweiliger Zugabe von Wasser wiederholt, der resultierende Niederschlag mit 500 ml Ethanol in einen Kolben gespült, durch Erwärmen auf 50°C (im Wasserbad) in Lösung und durch Einstellen in ein Eisbad zur Kristallisation gebracht. Nach scharfem Absaugen des Produktes erhält man 37,5 g (42,6 % der Theorie) schwach ethanolfeuchtes, beigefarbenes 3-Pentadecyl-2,4,6-trinitrophenol, Schmelzpunkt 53-56°C, DC: Kieselgel 60, Laufmittel: Ethylacetat/Methanol/Eisessig 90:5:5, R_{f} = 0,8.

Nach Trocknen der Substanz über Diphosphorpentoxid erhält man 35,75 g (40,1 % der Theorie) 3-Pentadecyl-2,4,6-trinitrophenol, Fp. 59-61°C.

### Beispiel 2

A) 2,4,6,8-Tetranitro-5-octadecyloxy-1-naphthol
   a) 5-Octadecyloxy-1-naphthol
      In einem 2 1 Dreihalskolben mit Claisenaufsatz, Thermometer, Calciumchloridrohr und Tropfrichter suspendiert man 40 g (0,25 mol) 1,5-Dihydroxynapthalin (Janssen 99 %) in 400 ml frisch destilliertem Dimethylformamid und gibt unter Rühren 6 g (0,25 mol) 97 % Natriumhydrid in kleinen Portionen innerhalb 40 Minuten zu. Dabei tritt neben Wasserstoffentwicklung und Temperaturanstieg auf 36°C Lösung Blaufärbung ein. Man rührt noch 30 Minuten nach und tropft in die noch 35°C warme Lösung innerhalb 10 Minuten 83,3 g (0,25 mol) 96% 1-Octadecylbromid zu. Danach rührt man 24 Stunden bei Raumtemperatur nach. Das angefallene Rohprodukt wird scharf abgesaugt und der Rückstand 15 Minuten mit 600 ml Wasser angerührt. Diese Prozedur wird nochmals wiederholt und der Filterrückstand solange mit Wasser (ca. 800 ml) gewaschen, bis das Filtrat farblos ist. Danach trocknet man den Filterkuchen bei 40°C im Trockenschrank über Diphosphorpentoxid. Man erhält 98,6 g hellbeige Kristalle vom Schmelzpunkt 76-78°C.
      Zur Weiterreinigung rührt man das Produkt dreimal mit je 750 ml Essigsäureethylester an, filtriert die ungelösten Anteile (40,8 g) hellbeige Kristalle ab, behandelt die Mutterlauge zweimal mit Kohle und engt im Vakuum ein. Man erhält 53,2 g (51,9 % der Theorie) beigefarbene Kristalle vom Schmelzpunkt 90-92°C. Dieses Produkt wird direkt für die Herstellung der tetranitrierten Verbindung (Beispiel 2 b) verwendet.
      DC, Kieselgel 60 (Merck), Laufmittel: Toluol/Methanol 50:1; R_{f} = 0,36.
   b) 2,4,6,8-Tetranitro-5-octadecyloxy-1-naphthol
      In einen 2 l Dreihalskolben mit großflächigem Rührer und Thermometer gibt man 1,2 l konz. Schwefelsäure, erwärmt auf 40°C und gibt 49,52 g (0,12 mol) 5-Octadecyloxy-1-naphthol möglichst rasch unter kräftigem Rühren zu. Nach 5-10 Minuten entsteht unter 2-3°C Temperaturanstieg ein dicker Kristallbrei. Man rührt dann 20 Minuten ohne Heizung nach, kühlt dann auf ca. 0°C ab und tropft Nitriersäure (hergestellt aus 34,9 ml Salpetersäure 65prozentig; unter Rühren und Kühlen bei ca. 10-20°C zu 70 ml konz. Schwefelsäure in ca. 15 Minuten gegeben) bei 0-5°C innerhalb 30 Minuten zu. Dabei färbt sich die Reaktionsmischung graubraun bis rotbraun. Nach vierstündigem Nachrühren bei 5-10°C wird auf ca. 5 kg Eis gegossen und das Rohprodukt 3 mal mit 2 l Essigsäurethylester extrahiert. Danach werden die Essigsäureethylesterphasen vereinigt, zweimal mit je 1 l Wasser gewaschen, die Essigsäureethylester-Phase über Natriumsulfat getrocknet, abgesaugt und eingeengt. Man erhält ca. 80 g dunkelbraunen harzigen Rückstand. Dieser wird säulenchromatographisch gereinigt. Man verwendet eine Säule von 7,5 cm innerem Durchmesser, Füllhöhe ca. 110 cm, Füllmittel: Kieselgel 60 (Merck), Laufmittel: Toluol/Aceton 5:2. Hauptfraktion R_{f} = 0,24.
      Diese Rohsubstanz wird nochmals mit ca. 400 ml Laufmittel angerührt, falls nicht alles gelöst, abgesaugt (Rückstand kann die Säule verstopfen) und das Filtrat auf die Säule gegeben und fraktionsweise eluiert. Man schneidet Fraktionen zu je 80 ml. Vorlauf (farbloses Eluat) ca. 2 l. Die Substanz enthaltenden Fraktionen (30-140) werden eingeengt. Man erhält 21 g rotbraunen zähen Brei, der nach längerem Stehen durchkristallisiert. Man löst dieses Produkt in 42 ml Aceton und fällt das Endprodukt durch langsame Zugabe der 5fachen Menge Isohexan bei Raumtemperatur. Nach fünfstündigem Rühren saugt man ab, wäscht den Filterkuchen mit Isohexan und trocknet im Vakuum über Diphosphorpentoxid und Molekularsieb bei Raumtemperatur. Man erhält 14,9 g (21 % der Theorie) des gewünschten Tetranitrooctadecyloxynaphthols, Fp. 236-238°C (Zers.). DC: Kieselgel 60 (Merck), Laufmittel: Methylenchlorid/Methanol 8:1; R_{f} = 0,27.
B) 2,4-Dinitro-5-octadecyloxy-1-naphthol
   In einem 250 ml Dreihalskolben mit Rührer und Thermometer tropft man zu einer Suspension von 8,25 g (0,02 Mol) 5-Octadecyloxy-1-naphthol in 40 ml Eisessig unter heftigem Rühren bei 20 bis 30 °C eine Mischung von 3,78 g (2,5 ml) (0,06 Mol) Salpetersäure (Dichte 1,52 g/cm³) und 20 ml Eisessig, rührt 2 Stunden bei 30 °C nach, saugt das abgeschiedene Rohprodukt über eine Glasfritte ab und wäscht den Rückstand mit wenig Isohexan. Nach Trocknen über Diphosphorpentoxid und Molekularsieb erhält man 6,4 g braune Kristalle. Dieses Material wird an einer Kieselgel 60 (E. Merck, Darmstadt, Deutschland) Säule (Durchmesser 4,5 cm, Füllhöhe 80 cm) chromatographisch gereinigt. Laufmittel: Toluol/Methanol = 49:1. Die entsprechenden Fraktionen werden eingeengt, wobei man 3,5 g orangerote Kristalle erhält. Nach Umkristallisieren aus 50 ml n-Heptan werden 2,82 g (27,1 % der Theorie) der gewünschten Dinitroverbindung als hellbraune Kristalle, Fp. 87 - 89 °C erhalten. DC: Kieselgel 60 (E. Merck, Darmstadt, Deutschland), Laufmittel: Toluol/Methanol 30:1, Rf = 0,66.

### Beispiel 3

### [(2,6-(Dichlor)-4-(methylsulfonyl)-phenyl)-hydrazono]propandinitril

Man löst 2,1 g (0.03 mol) Natriumnitrit in 30 ml konz. Schwefelsäure. Dabei steigt die Temperatur auf 50°C an. Man kühlt auf 20°C ab, tropft 20 ml Eisessig bei 15 - 20°C zu und gibt 7,2 g (0,03 mol) 2,6-Dichlor-4-(methylsulfonyl)anilin portionsweise zu und rührt noch eine Stunde bei 20°C nach.

Man löst 1,98 g Malondinitril in 75 ml Ethanol, gibt eine Lösung von 74 g Natriumacetat-trihydrat in 35 ml Wasser zu und tropft unter Rühren bei 19°C die vorstehend hergestellte Diazoniumsalzlösung zu. Nach einstündigem Nachrühren wird das gebildete Kristallisat abgesaugt und der Filterrückstand in 300 ml Wasser eingetragen, mit Methylenchlorid extrahiert, 200 ml Trichlorethan zugegeben, die Extrakte über Natriumsulfat getrocknet und auf ca. 100 ml eingeengt. Ausgefallene Kristalle werden abgesaugt und getrocknet. Man erhält 7,12 g (74,8 % der Theorie) sandfarbene Kristalle, Fp. 165-168°C.

### Beispiel 4

Analog Beispiel 3 werden die folgenden Propandinitrilhydrazone hergestellt
a) [(2,3,5,6-Tetrafluorphenyl)-hydrazono]propandinitril,
   Fp. 103-106°C,
   aus 2,3,5,6-Tetrafluoranilin
b) [(4-Nitrophenyl)-hydrazono]propandinitril,
   Fp. 220°C
   aus p-Nitroanilin
   (Lithgoe, Todd, Topham, Chem. Soc. 1944, 315)
c) [(2,4-Dinitrophenyl)-hydrazono]propandinitril,
   DC, Kieselgel 60 (Merck), Laufmittel: Methylenchlorid/Methanol = 98:2, R_{f} = 0,28
   aus 2,4-Dinitroanilin (NL-A-6411189)
d) [(2,4-Dichlorphenyl)-hydrazono]propandinitril,
   Fp 114-116°C
   aus 2,4-Dichloranilin (NL-A-6411189)
e) [(3,5-Dichlorphenyl)-hydrazono]propandinitril,
   Fp 200°C (Zers.),
   aus 3,5-Dichloranilin (NL-A-6411189)
f) [(3,5-Dichlor-2,4,6-tribromphenyl)-hydrazono]propandinitril,
   Fp 193-196°C,
   aus (3,5-Dichlor-2,4,6-tribromanilin
   (NL-A-6411189)
g) [(2,4-Dichlor-6-bromphenyl)-hydrazono]propandinitril,
   Fp 134-136°C
   aus 2,4-Dichlor-6-bromanilin (Eur.J.Med. Chem. Clin. Therap. 12, 361 [1977])
h) [(2,4,6-Trichlorphenyl)-hydrazono]propandinitril,
   Fp 225-226°C,
   aus 2,4,6-Trichloranilin (Eur.J.Med.Chem.Clin.
   Therap. 12, 361 [1977])
i) [(2-Trifluormethyl-4-nitrophenyl)-hydrazono]propandinitril,
   Fp 245°C,
   aus 2-Trifluormethyl-4-nitroanilin
j) [(2,4,6-Tribromphenyl)-hydrazono]propandinitril,
   Fp 153°C,
   aus 2,4,6,-Tribromanilin (Eur.J.Med.Chem.Clin.
   Therap. 12, 361 [1977])
k) [(2-Methansulfonyl-4-nitrophenyl)-hydrazono]propandinitril,
   Fp 220°C,
   aus 2-Methansulfonyl-4-nitroanilin
l) [(2,4-Dinitro-6-cyanophenyl)-hydrazono]propandinitril,
   DC, Kieselgel 60 (Merck), Laufmittel: Toluol/Methylethylketon 1:2, R_{f} = 0,50,
   aus 2,4-Dinitro-6-cyanoanilin (W. Thiel et al., J. Pract. Chem. 328, 499 (1986))
m) [(3,5-Di-{trifluormethyl}phenyl)-hydrazono]propandinitril,
   Fp 150-151°C,
   aus 3,5-Di-(trifluormethyl)-anilin

### Beispiel 5

### 4-[(2,6-Dibrom-4-nitrophenyl)azo]-2-octadecyloxy-1-naphthol

a) 2-Octadecyloxynaphthalin
   Man gibt in einen 4 l-Dreihalskolben mit Rührer, Kühler und Thermometer 172,8 g (1,2 mol) 2-Naphthol (98 %) zu einer Lösung von 48 g (1,2 mol) Natriumhydroxid (99 %) in 1 l Ethanol, fügt nach erfolgter Lösung 417 g (1,25 mol) n-Octadecylbromid hinzu und erhitzt die Reaktionsmischung 14 Stunden unter Rückfluß. Nach Zugabe von weiteren 1 l Ethanol wird die heiße Lösung zur Entfernung anorganischen Materials über ein Seitzfilter abgesaugt und das schwach rosa gefärbte Filtrat durch 30minütiges Einstellen in ein Eisbad zur Kristallisation gebracht. Nach Absaugen der fast farblosen Kristalle wäscht man den Filterkuchen portionsweise mit ca. 700 ml Ethanol und erhält nach Trocknen über Diphosphorpentoxid 371,9 g (93,7 % der Theorie) 2-Octadecyloxynaphthalin, farblose Kristalle, Fp. 64-68°c.
   DC: Kieselgel 60 (Merck), Laufmittel: n-Heptan/Methylethylketon 2:1, R_{f} = 0,34
b) 2-Octadecyloxy-1-naphthol
   In einem 10 l-Dreihalskolben mit Rührer, Claisenaufsatz Thermometer und Kühler mit Chlorcalciumrohr gibt man 594 g (1,5 mol) 2-Octadecyloxynaphthalin und 397 g (0,75 mol) Bleitetraacetat in ein Gemisch von 3 l Eisessig und 600 ml Acetanhydrid und erwärmt auf 55°C. Während 4 Tagen werden im Zeitabstand von 24 Stunden weitere 400 g (jeweils 100 g) Bleitetraacetat portionsweise unter Rühren zugegeben. Danach wird die entstandene gelbe Lösung auf Raumtemperatur abgekühlt, nach Zugabe von 1,5 l Wasser 30 Minuten nachgerührt, der entstandene Kristallbrei abgesaugt und portionsweise mit 2 l Wasser gewaschen. Das feuchte Rohprodukt wird in 4 l Toluol gelöst und dreimal mit je 1 l Wasser, dreimal mit 1 l gesättigter Natriumhydrogencarbonatlösung und dann abermals 3 mal mit 1 l Wasser durchgeschüttelt. Nach Trocknen der Toluolphase über Natriumsulfat, Absaugen und Einengen erhält man 635 g braunes Rohprodukt, das chromatographisch wie folgt gereinigt wird: Man löst das erhaltene Kristallisat in einer Mischung von 1,3 l Toluol/Isohexan 5:2 und gibt die Lösung auf eine Kieselgel 60 (Merck)-säule, innerer Durchmesser 11,5 cm, Füllhöhe 1,2 m. Als Laufmittel verwendet man Toluol/Isohexan 5:2 und schneidet Fraktionen von ca. 300 ml. Die Fraktionen 9-52 werden vereinigt und bis zur Gewichtskonstanz eingeengt. Man erhält 324,2 g 2-Octadecyloxy-1-naphtholacetat, Fp. 67-68°C. Dieses wird ohne weitere Reinigung in 1,8 l Methanol unter Erwärmen gelöst und auf 20°C abgekühlt. Zu der entstandenen Suspension tropft man innerhalb 15 Minuten ohne Kühlung unter Rühren 93 ml konz. Schwefelsäure zu, wobei die Temperatur auf 35°C ansteigt. Anschließend erhitzt man 2 Stunden unter Rückfluß, kühlt dann mit einem Eisbad und rührt 30 Minuten unter Eiskühlung nach. Die gebildeten Kristalle werden abgesaugt, mit 150 ml eiskaltem Methanol gewaschen und bei 35 °C im Trockenschrank über Diphosphorpentoxid getrocknet. Man erhält 294,4 (47,5 % der Theorie) 2-Octadecyloxy- 1-naphthol, farblose Kristalle, Fp. 58-59°C.
c) 4-[(2,6- Dibrom-4-nitrophenyl)azo]-2-octadecyloxy-1-naphthol
   In einem 2 1 Dreihalskolben mit Rührer, Claisenaufsatz und Thermometer werden 22,7 g (0,33 mol) Natriumnitrit während 10-15 Minuten unter Rühren in 300 ml konz. Schwefelsäure eingetragen, wobei man die Temperatur der Reaktionslösung auf 35°C ansteigen läßt. Man kühlt danach auf 20°C und tropft in ca. 15-20 Minuten 230 ml Eisessig so zu, daß die Temperatur unter Eiskühlung bei 20-25°C gehalten wird. Danach gibt man 97,6 ml (0,33 mol) 2,6-Dibrom-4-nitroanilin (Riedel de Haen [99 %GC]) portionsweise während 10 Minuten unter gelegentlichem Kühlen zu, wobei die Temperatur auf19-21°C gehalten wird und rührt noch 3 Stunden nach. Danach gießt man auf 3,5 l Eiswasser und gibt die entstandene Diazoniumsalzlösung rasch zu einer Lösung von 124 g (0,3 mol) 2-Octadecyloxy-1-naphthol in einer Mischung von 3 l Eisessig und 300 ml Chloroform unter Zugabe von 180 g (1,33 mol) Natriumacetat-trihydrat. (Bei der Herstellung der Lösung des Naphtholethers ist darauf zu achten, daß nach Eintragen desselben in Eisessig-Chloroform unter Zugabe von Natriumacetat nach einem Temperaturanstieg auf ca. 45°C wieder auf 20°C abgekühlt wird.) Nach 3 Stunden Rühren im Eisbad wird das erzeugte Kristallisat abgesaugt, der Rückstand dreimal mit je 500 ml Wasser gewaschen und im Trockenschrank bei 40°C getrocknet. Das Rohprodukt - 295,5 g hellbraune Kristalle - werden chromatographisch gereinigt. Die Azoverbindung wird in 1 l Toluol/Methylenchlorid 2:5 gelöst auf eine Kieselgel 60 (Merck)-säule mit innerem Durchmesser 11,5 cm, Füllhöhe 1,2 m aufgetragen und mit Toluol/Methylenchlorid 2:5 eluiert. Man schneidet Fraktionen von ca. 70 ml. Die Fraktionen 57-173 werden vereinigt und eingeengt. Man erhält 134,2 g braune Kristalle. Diese werden in 480 ml Toluol bei 80°C gelöst, auf 65°C gekühlt und unter kräftigem Rühren mit 800 ml Isohexan versetzt. Man läßt unter Rühren auf 20°C abkühlen, stellt über Nacht in den Kühlschrank, saugt die gebildeten Kristalle ab und wäscht den Filterköcher 2 mal mit 300 ml eiskaltem Toluol/Isohexan 1:1,3 und anschließend mit 300 ml Isohexan. Anschließend trocknet man im Trockenschrank bei 40°C über Diphosphorpentoxid bis zur Gewichtskonstanz. Man erhält 119,9 g (55,5 % der Theorie) Azoverbindung, hellbraune Kristalle,
   Fp. 102-103°C
   DC, Kieselgel 60 (Merck), Laufmittel: Toluol-Methylenchlorid 2:5, Rf = 0,37.

### Beispiel 6

### 4-[(2-Brom-4-nitro-6-trifluormethylphenyl)-azo]-2-octadecyloxy-1-naphthol

### Wird analog Beispiel 5 hergestellt

aus 2-Brom-4-nitro-6-trifluormethylanilin (M. Hauptschein et al., J. Amer. Chem. Soc. 76, 1051 (1954)),
Fp. 84 °C.

### Beispiel 7

a) 2-(3,7,11,15-Tetramethyl-2-hexadecenyl)-3-methyl-4-(2,4-dinitrophenyl)azo]-1-naphthol
   In einem 2 1 Dreihalskolben mit Rührer, Kühler und Thermometer suspendiert man 19,8 g (0.1 mol) 2,4-Dinitrophenylhydrazin in 400 ml Ethanol unter Zugabe von 9 ml (0,11 mol) konz. Salzsäure und gibt 45 g (0,1 mol) Vitamin K₁ [2-Methyl-3-(3,7,11,15-tetramethyl-2-hexadecyl)-1,4-naphthochinon] zu, rührt 15 Minuten bei Raumtemperatur, dann erhitzt man 4 Stunden unter Rückfluß. Danach wird im Vakuum eingeengt. Man erhält 64 g rotbraune viskose Masse. Diese wird an einer Kieselgel-60-(Merck)-säule, innerer Durchmesser 10,5 cm, Füllhöhe 110 cm mit Methylenchlorid/n-Heptan als Laufmittel chromatographisch gereinigt. Wegen der Schwerlöslichkeit des Reaktionsproduktes wird das Rohprodukt in 350 ml des Laufmittels gelöst, unlösliche Anteile über ein Seitzfilter abfiltriert und auf die Kieselgelsäule aufgetragen. Die einschlägigen Chargen werden vereinigt, im Vakuum eingeengt und das organgefarbene, wachsartige Produkt zweimal aus je 100 ml n-Propanol/Ligroin 1:1 umkristallisiert, der Rückstand 2 mal mit 20 ml n-Propanol/Ligroin 1:1 gewaschen und bis zur Gewichtskonstanz getrocknet. Man erhält 19,41 g (31 % der Theorie) orangefarbene, wachsartige, DC-einheitliche Kristalle, Fp. 110°c.
   Analog kann hergestellt werden:
b) 2-(3,7,11,15-Tetramethyl-2-hexadecenyl)-3-methyl-4-(4-nitrophenyl)azo]-1-naphthol
   DC, Kieselgel 60 (Merck), Laufmittel: Toluol/Methanol = 50:1;
   R_{f} = 0,22
   aus 4-Nitrophenylhydrazin

### Beispiel 8

### Allgemeine Herstellvorschrift für Testträger

Zur Herstellung eines Testträgers gemäß Fig. 1 wird durchsichtige Polyesterfolie (200 µm dick) mit den in den nachfolgenden Beispielen genannten Mischungen beschichtet und getrocknet. Die beschichtete Folie wird in 15 mm breite Streifen geschnitten und mittels Schmelzkleber als Schicht (1) längs auf 150 mm breite weiße Polyesterfolie (5) geklebt. Ebenso werden Streifen von Glasfaservlies mit einem Flächengewicht von 30 g/m² als Transportschicht (2), Glasfaservlies mit einem Flächengewicht von 60 g/m² als Abtrennschicht (3) und Polyamidgewebe als Schutzschicht (4) längs auf diese weiße Polyesterfolie geklebt, so daß nach Querschneiden 6 mm breite Teststreifen gemäß Fig. 1 entstehen.

Testträger gemäß Fig. 2 werden analog hergestellt. Die Schicht (11) besteht aus Filterpapier, das mit Puffersubstanz imprägniert ist.

Die erfindungsgemäße Filmschicht bzw. Testträger werden so eingesetzt, daß auf das Polyamidgewebe (4) 30 µl der zu untersuchenden Probe aufgebracht und der Testträger danach in das handelsübliche Reflexionsphotometer Reflotron® (Boehringer Mannheim GmbH, Mannheim, Bundesrepublik Deutschland) eingeführt wird. Die Flüssigkeit dringt in das Glasfaservlies (3) ein, wo im Falle von Vollblut die Erythrozyten abgetrennt werden und gelangt in die als Transportschicht dienende Glasfaserzone (8). Im Reflexionsphotometer wird der Film unter der Klappe (1) bzw. (10) durch Druck auf die Klappe mit der Flüssigkeit in der Transportschicht (2) in Berührung gebracht und die entstehende Farbe wird bei 642 nm und 37 °C reflexionsphotometrisch gemessen.

### Beispiel 9

Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-vinyllaurat-Copolymer (Vinnapas® B 500/20 VL, Wacker Chemie, München, Bundesrepublik Deutschland) | 13,11 g |
| 2,2-Diphenyl-1-cyano-acrylsäure-ätylhexylester (Uvinul® N539, BASF, Ludwigshafen, Deutschland) | 16,04 g |
| 4-[(2,6-Dibrom-4-nitrophenyl)azo]-2-octadecyloxy-1-naphthol (Herstellung gemäß Beispiel 5) | 0,173 g |
| 2,4,6,8-Tetranitro-5-octadecyloxy-naphthol-1 (Beispiel 2) | 0,0456 g |
| Valinomycin | 0,2673 g |
| Diatomeenerde (Celatom® MW 25, Eagle-Picher, Cincinatti, USA ) | 25,13 g |
| Essigsäurebutylester | 45,17 g |

Auf diese Schicht wird mit einer Naßfilmdicke von 150 µm eine zweite Schicht folgender Zusammensetzung aufgebracht und gleichfalls getrocknet:

| | |
|---|---|
| Hydroxyäthylcellulose (Natrosol® 250G, Hercules Inc., Willmington, Dellaware, USA), 2 % in Wasser | 24 g |
| N,N-Bis-(hydroxyäthyl)-aminoäthansulfonsäure (BES) | 8,2 g |
| Ethanol | 42 ml |
| mit LiOH auf pH 7,5 eingestellt | |

Des weiteren wird ein Testfilm der gleichen Zusammensetzung, nur ohne 2,4,6,8-Tetranitro-5-octadecyloxy-naphthol-1 hergestellt.

Aus den beschichteten Folien werden wie in Beispiel 8 beschrieben, Teststreifen gemäß Fig. 1 hergestellt und vermessen. Die Messung erfolgt 60 Sekunden nach Berührung der Probe mit dem Reagenzfilm.

Bei der Verwendung von Seren mit verschiedenen Gehalten an Kalium wird folgende Abhängigkeit der Reflektion (%R) vom Kaliumgehalt gefunden:

**Tabelle 2:**

| Kaliumgehalt [mmol Kalium/l] | Reflektion [%R] | |
|---|---|---|
| | mit | ohne |
| | 2,4,6,8-Tetranitro-5-octadecyloxy-naphthol-1 | |
| 0,24 | 62,0 | 34,4 |
| 1,09 | 55,8 | 28,5 |
| 1,87 | 50,3 | 24,8 |
| 3,18 | 43,2 | 21,2 |
| 4,15 | 38,3 | 19,3 |
| 6,08 | 32,3 | 16,8 |
| 8,10 | 27,5 | 15,0 |
| 10,22 | 24,0 | 13,8 |
| 12,10 | 21,5 | 12,9 |

Wie man sieht, sind im diagnostisch wichtigen Bereich von ca. 2-6 mmol/l Kalium mit der erfindungsgemäßen Säure eine Meßwertdifferenz von ca. 18 %R zu erzielen, während es ohne diese Säure nur ca. 8 %R sind.

### Beispiel 10

Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-Maleinsäuredibutylester-Copolymer (Mowilith® 35/73, Hoechst, Frankfurt, Deutschland) | 14,7 g |
| 2,2-Diphenyl-1-cyano-acrylsäure-ätylhexylester (Uvinul® N539, BASF, Ludwigshafen, Deutschland) | 18,4 g |
| 4-[(2,6-Dibrom-4-nitrophenyl)azo]-2-octadecyloxy-1-naphthol (Herstellung gemäß Beispiel 5) | 0,130 g |
| Bis-(2-hydroxy-3,5,6-trichlorphenyl)-methan (Hexachlorophen, Aldrich, Steinheim, Deutschland) | 0,029 g |
| Valinomycin | 0,600 g |
| Diatomeenerde (Celatom® MW 25, Eagle-Picher, Cincinatti, USA) | 28,2 g |
| Essigsäurebutylester | 50,7 g |

Auf diese Schicht wird mit einer Naßfilmdicke von 150 µm eine zweite Schicht folgender Zusammensetzung aufgebracht und gleichfalls getrocknet:

| | |
|---|---|
| Hydroxyäthylcellulose (Natrosol® 250G, Hercules Inc., Willmington, Delaware, USA), 4 % in Wasser | 41,5 g |
| N,N-Bis-(hydroxyäthyl)-aminoäthansulfonsäure (BES) | 8,5 g |
| Ethanol | 64 ml |
| mit LiOH auf pH 7,8 eingestellt | |

Des weiteren wird ein Testfilm mit der gleichen Zusammensetzung, nur ohne Hexachlorophen hergestellt.

Aus den beschichteten Folien werden wie in Beispiel 8 beschrieben, Teststreifen gemäß Fig. 1 hergestellt und vermessen. Die Messung erfolgt 60 Sekunden nach Berührung der Probe mit dem Reagenzfilm.

Bei der Verwendung von Seren mit verschiedenen Gehalten an Kalium wird folgende Abhängigkeit der Reflektion (%R) vom Kaliumgehalt gefunden:

**Tabelle 3**

| Kaliumgehalt [mmol Kalium/l] | Reflektion [%R] | |
|---|---|---|
| | mit | ohne |
| | Hexachlorophen | |
| 1,00 | 54,2 | 33,4 |
| 1,98 | 47,8 | 28,5 |
| 2,99 | 41,6 | 25,1 |
| 4,12 | 37,4 | 22,7 |
| 6,00 | 30,8 | 19,3 |
| 8,04 | 26,4 | 17,1 |
| 10,12 | 23,2 | 15,4 |
| 11,98 | 21,7 | 14,3 |

Wie man sieht, sind im diagnostisch wichtigen Bereich von ca. 2-6 mmol/1 Kalium mit der erfindungsgemäßen Säure eine Meßwertdifferenz von ca. 17 %R zu erzielen, während es ohne diese Säure nur ca. 9 %R sind.

### Beispiel 11

Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-vinyllaurat-Copolymer (Vinnapas® B 500/20 VL, Wacker Chemie, München, Bundesrepublik Deutschland) | 19,6 g |
| | |
| 2,2-Diphenyl-1-cyano-acrylsäure-ätylhexylester (Uvinul® N539, BASF, Ludwigshafen, Deutschland) | 24,0 g |
| 4-[(2-Brom-4-nitro-6-trifluormethylphenyl)-azo]- 2-octadecyloxy-1-naphthol (Herstellung gemäß Beispiel 6) | 0,071 g |
| [(2,4-Dinitrophenyl)-hydrazono]propandinitril (Beispiel 4 c) | 0,052 g |
| Valinomycin | 0,30 g |
| Diatomeenerde (Celatom® MW 25, Eagle-Picher, Cincinatti, USA) | 37,5 g |
| m-Xylol | 67,4 g |

Des weiteren wird ein Testfilm der gleichen Zusammensetzung, nur ohne [(2,4-Dinitrophenyl)-hydrazono]propandinitril hergestellt.

Langfaserpapier 6776 (Schöller und Hösch, Gernsbach, Deutschland) wird mit folgender Lösung imprägniert und getrocknet:

| | |
|---|---|
| N,N-Bis-(hydroxyäthyl)-aminoäthansulfonsäure (BES) | 8,5 g |
| n-Octylglucosid | 0,1 g |
| Wasser, dest. | 91,5 ml |
| mit LiOH auf pH 7,5 eingestellt | |

Aus den beschichteten Folien (10) und dem Pufferpapier (11) werden wie in Beispiel 8 beschrieben, Teststreifen gemäß Fig. 2 hergestellt und vermessen. Die Messung erfolgt 60 Sekunden nach Berührung der Probe mit dem Reagenzfilm.

Bei der Verwendung von Seren mit verschiedenen Gehalten an Kalium wird folgende Abhängigkeit der Reflektion (%R) vom Kaliumgehalt gefunden:

**Tabelle 4**

| Kaliumgehalt [mmol Kalium/l] | Reflektion [%R] | |
|---|---|---|
| | mit | ohne |
| | [(2,4-Dinitrophenyl)-hydrazono]propandinitril | |
| 0,08 | 63,6 | 33,9 |
| 1,01 | 57,0 | 24,8 |
| 1,98 | 46,3 | 21,2 |
| 3,10 | 37,6 | 18,9 |
| 4,08 | 31,0 | 17,6 |
| 6,08 | 25,0 | 15,7 |
| 8,05 | 22,0 | 14,5 |
| 9,90 | 20,0 | 13,6 |

Wie man sieht, ist im diagnostisch wichtigen Bereich von ca. 2 - 6 mmol/l Kalium mit der erfindungsgemäßen Säure eine Meßwertdifferenz von ca. 21 %R zu erzielen, während es ohne diese Säure nur ca. 5,5 %R sind.

Teststreifen mit einer ähnlichen Remissionsdifferenz erhält man, wenn man anstelle des 2,4-Dinitriphenylhydrazons das 3,5-Di-trifluormethyl-phenylhydrazon des Mesoxalsäuredinitrils (Beispiel 4 m) verwendet.

### Beispiel 12

Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-vinyllaurat-Copolymer (Vinnapas® B 500/20 VL, Wacker Chemie, München, Deutschland) | 5,9 g |
| 2,2-Diphenyl-1-cyano-acrylsäure- ätylhexylester (Uvinul® N539, BASF; Ludwigshafen, Bundesrepublik Deutschland) | 7,2 g |
| 2-(3,7,11,15-Tetramethyl-2-hexadecenyl)-3- methyl-4-(4-nitrophenyl)azo]-1-naphthol (Herstellung gemäß Beispiel 7) | 0,032 g |
| 2,4,6-Trinitro-3-pentadecyl-phenol (Beispiel 1) | 0,020 g |
| Valinomycin | 0,120 g |
| Diatomeenerde (Celatom® MW 25, Eagle-Picher, Cincinatti, USA) | 11,3 g |
| Essigsäurebutylester | 20,3 g |

Auf diese Schicht wird mit einer Naßfilmdicke von 150 µm eine zweite Schicht folgender Zusammensetzung aufgebracht und gleichfalls getrocknet:

| | |
|---|---|
| Hydroxyäthylcellulose (Natrosol® 250G, Hercules Inc., Willmington, Delaware, USA), 2 % in Wasser | 150 g |
| Borsäure | 4,64 g |
| Ethanol | 198 ml |
| mit LiOH auf pH 9,5 eingestellt | |

Des weiteren wird ein Testfilm der gleichen Zusammensetzung, nur ohne 2,4,6-Trinitro-3-pentadecyl-phenol hergestellt.

Aus den beschichteten Folien werden, wie in Beispiel 8 beschrieben, Teststreifen gemäß Fig. 1 hergestellt und vermessen. Die Messung erfolgt 60 Sekunden nach Berührung der Probe mit dem erfindungsgemäßen Reagenz film.

Mit Seren mit verschiedenen Gehalten an Kalium werden folgende Reflektionswerte gemessen:

**Tabelle 5**

| Kaliumgehalt [mmol Kalium/l] | Reflektion [%R] | |
|---|---|---|
| | mit | ohne |
| | 2,4,6-Trinitro-3-pentadecyl-phenol | |
| 0,24 | 66,5 | 54,3 |
| 1,09 | 62,8 | 48,4 |
| 1,87 | 59,9 | 44,2 |
| 3,18 | 55,8 | 39,8 |
| 4,15 | 52,7 | 37,7 |
| 6,08 | 47,8 | 33,9 |
| 8,10 | 43,6 | 31,3 |
| 10,22 | 40,1 | 29,0 |
| 12,10 | 37,4 | 27,1 |

Wie man sieht, ist im diagnostisch wichtigen Bereich von ca. 2 - 6 mmol/l Kalium mit der erfindungsgemäßen Säure eine Meßwertdifferenz von ca. 12 %R zu erzielen, während es ohne diese Säure nur ca. 10 %R sind.

Mit Teststreifen analoger Zusammensetzung, die anstelle von 2,4,6-Trinitro-3-pentadecyl-phenol äquimolare Mengen an 2,4-Dinitro-5-octadecyloxy-naphthol-1 (Herstellung Beispiel 2B) enthalten, werden ähnliche Ergebnisse mit einer Meßwertdifferenz von ca. 11,5 % erhalten.

## Patentansprüche

1. Verfahren zur Bestimmung eines Ions in einer wässrigen Flüssigkeit, bei dem das Ion durch einen Ionophor in eine mit der wässrigen Flüssigkeit nicht mischbare Phase gelangt und ein dort anwesender pH-Indikator dadurch eine Farbänderung erfährt, welche zur Bestimmung des Ions herangezogen wird, dadurch gekennzeichnet, daß zur Erhöhung der Meßempfindlichkeit in der mit der wässrigen Flüssigkeit nicht mischbaren Phase eine Substanz aus der Gruppe der Verbindungen der allgemeinen Formel I 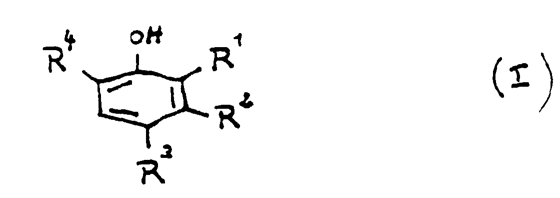 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 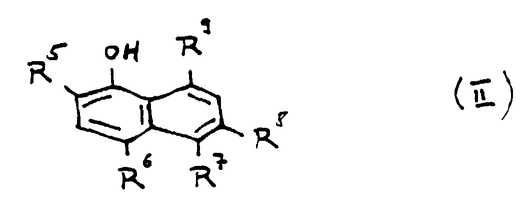 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R8 und R⁹ auch Wasserstoff bedeuten können und
der allgemeinen Formel III 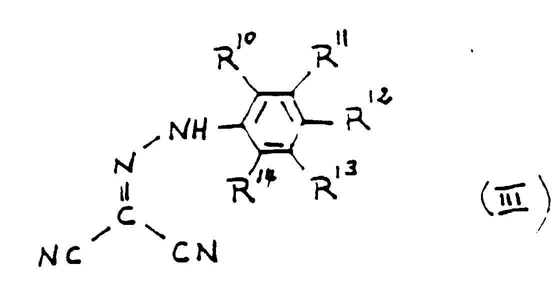 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
eingesetzt wird.

2. Verfahren gemäß Anspruch 1, dadurch gekennzeichnet, daß als pH-Indikator ein Naphtholderivat der allgemeinen Formel IV 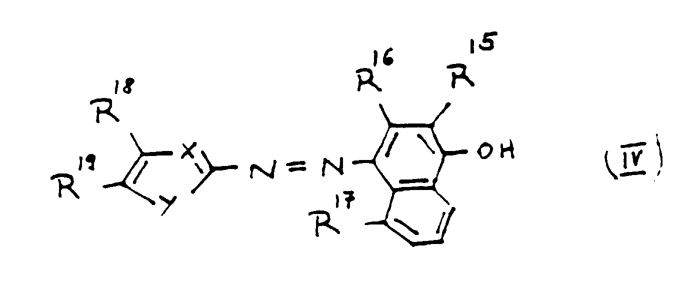 in der
R¹⁵, R¹⁶, R¹⁷ gleich oder verschieden sind und jeweils Wasserstoff, eine Alkyl- oder Alkoxygruppe darstellen, wobei mindestens einer der Reste ein (C₈-C³⁰)-Alkyl- oder Alkoxyrest ist,
R¹⁸ Wasserstoff oder eine Alkylgruppe,
R¹⁹ eine Nitrogruppe, eine durch Halogen substituierte Alkylgruppe, eine Cyanogruppe, eine Sulfonamidgruppe oder eine Alkylsulfonylgruppe,
X Stickstoff oder den Rest CR²⁰ und
Y Schwefel oder den Rest CR²¹ = CR²²,
wobei R²⁰, R²¹, R²² gleich oder verschieden sind und jeweils Wasserstoff, Halogen, eine Nitrogruppe, eine Cyanogruppe, eine Alkyl- oder durch Halogen substituierte Alkylgruppe oder eine Alkylsulfonylgruppe bedeuten,
eingesetzt wird.

3. Verwendung einer Substanz aus der Gruppe der Verbindungen der allgemeinen Formel I 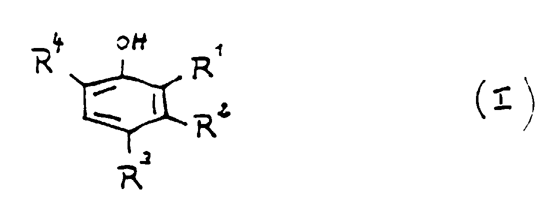 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 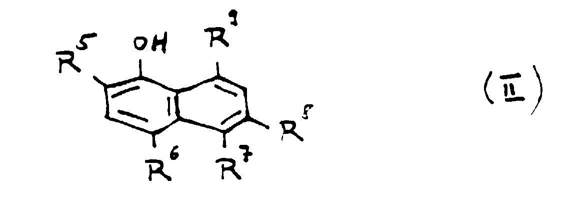 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R8 und R⁹ auch Wasserstoff bedeuten können und
der allgemeinen Formel III 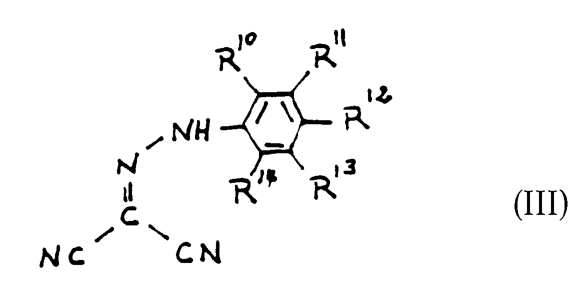 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten
zur Erhöhung der Meßempfindlichkeit bei Verfahren zur Bestimmung von Ionen, bei denen die Ionen durch Ionophore in eine mit der wässrigen Flüssigkeit nicht mischbare Phase gelangen und ein dort anwesender pH-Indikator dadurch eine Farbänderung erfährt, welche zur Bestimmung der Ionen herangezogen wird.

4. Mittel zur Bestimmung eines Ions in einer wässrigen Flüssigkeit, enthaltend einen Ionophor und einen pH-Indikator in einem mit Wasser nicht mischbaren Medium, dadurch gekennzeichnet, daß es zur Erhöhung der Meßempfindlichkeit eine Substanz aus der Gruppe der Verbindungen der allgemeinen Formel I 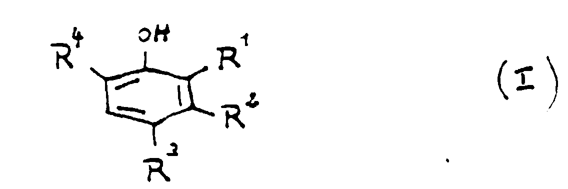 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 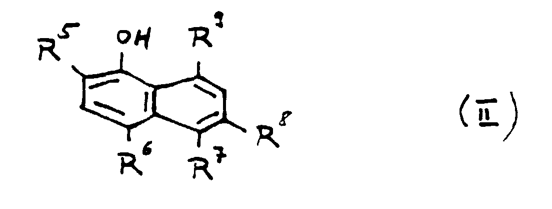 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R8 und R⁹ auch Wasserstoff bedeuten können und
der allgemeinen Formel III 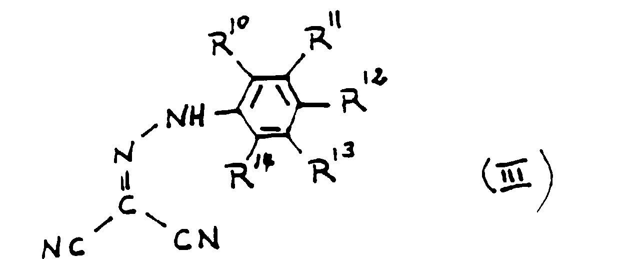 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
enthält.

5. Mittel gemäß Anspruch 4, dadurch gekennzeichnet, daß es als pH-Indikator ein Naphtholderivat der allgemeinen Formel IV 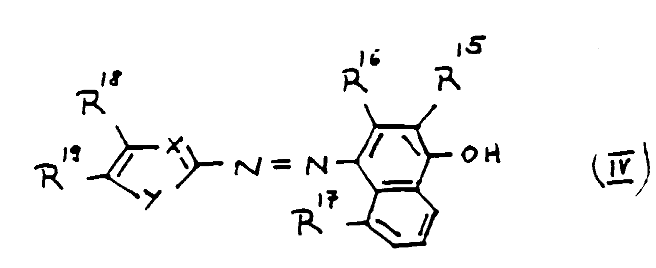 in der
R¹⁵, R¹⁶, R¹⁷ gleich oder verschieden sind und jeweils Wasserstoff, eine Alkyl- oder Alkoxygruppedarstellen, wobei mindestens einer der Reste ein (C₈-C₃₀)-Alkyl- oder Alkoxyrest ist,
R¹⁸ Wasserstoff oder eine Alkylgruppe,
R¹⁹ eine Nitrogruppe, eine durch Halogen substituierte Alkylgruppe, eine Cyanogruppe, eine Sulfonamidgruppe oder eine Alkylsulfonylgruppe,
X Stickstoff oder den Rest CR²⁰ und
Y Schwefel oder den Rest CR²¹ = CR²²,
wobei R²⁰, R²¹, R²² gleich oder verschieden sind und jeweils Wasserstoff, Halogen, eine Nitrogruppe, eine Cyanogruppe, eine Alkyl- oder durch Halogen substituierte Alkylgruppe oder eine Alkylsulfonylgruppe bedeuten,
enthält.

6. Verwendung einer Substanz aus der Gruppe der Verbindungen der allgemeinen Formel I 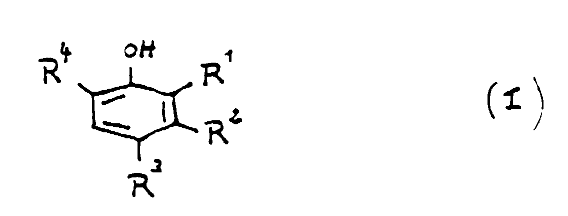 in der
einer der Reste R¹ bis R⁴ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten,
der allgemeinen Formel II 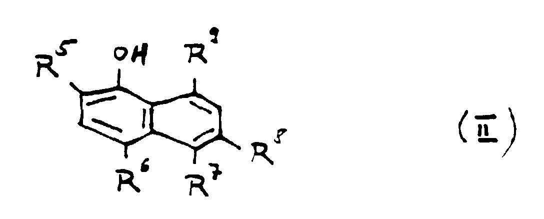 in der
einer der Reste R⁵ bis R⁹ ein Rest aus der Gruppe der Alkylreste, Alkoxyreste und Aralkylreste ist und
die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können und
der allgemeinen Formel III 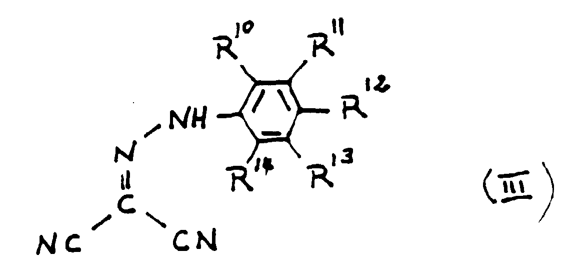 in der
R¹⁰, R¹¹, R¹², R¹³, R¹⁴ gleich oder verschieden sind und jeweils Wasserstoff, eine Nitrogruppe, Halogen, eine Cyanogruppe, eine Alkylsulfonylgruppe oder eine mit Halogen substituierte Alkylgruppe bedeuten,
zur Herstellung eines Mittels zur Bestimmung eines Ions, das einen Ionophor und einen pH-Indikator in einem mit Wasser nicht mischbaren Medium enthält.

7. Verbindung der allgemeinen Formel II 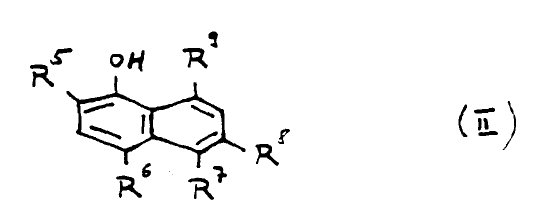 in der
R⁷ einen Alkyl-, Alkoxy- oder Aralkylrest darstellt und die übrigen Reste gleich oder verschieden sind und jeweils eine Nitrogruppe, Halogen, eine Cyanogruppe, eine Alkylsulfonylgruppe oder eine mit Halogen substituierte Alkylgruppe bedeuten oder wenn R⁵ und R⁶ Nitrogruppen darstellen, R⁸ und R⁹ auch Wasserstoff bedeuten können.

8. Verbindung aus der Gruppe der Substanzen
[(2,3,5,6-Tetrafluorphenyl)-hydrazono]propandinitril,
[(2-Trifluormethyl-4-nitrophenyl)-hydrazono]propandinitril,
[(2-Methansulfonyl-4-nitrophenyl)-hydrazono]propandinitril,
[(2,4-Dinitro-6-cyanophenyl)-hydrazono]propandinitril und
[(3,5-Di-(trifluormethyl)phenyl)-hydrazono]propandinitril.

## Claims

1. Method for the determination of an ion in an aqueous liquid in which the ion passes by means of an ionophore into a phase which is immiscible with the aqueous liquid and as a result a pH indicator which is present there undergoes a colour change which is used for the determination of the ion, wherein in order to increase the sensitivity of the measurement a substance from the group of compounds having the general formula I 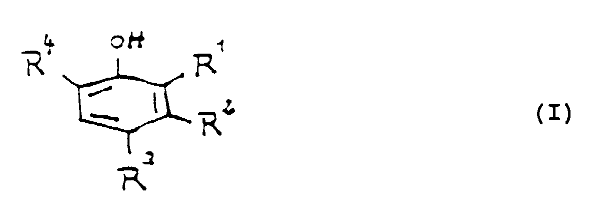 in which
one of the residues R¹ to R⁴ is a residue from the group of alkyl residues, alkoxy residues and aralkyl residues and
the other residues are the same or different and each denotes a nitro group, halogen, a cyano group, an alkylsulfonyl group or an alkyl group substituted with halogen,
having the general formula II 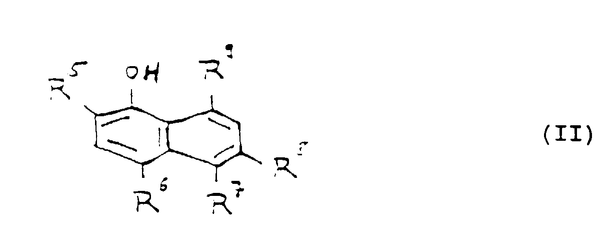 in which
one of the residues R⁵ to R⁹ is a residue from the group of alkyl residues, alkoxy residues and aralkyl residues and
the other residues are the same or different and each denotes a nitro group, halogen, a cyano group, an alkylsulfonyl group or an alkyl group substituted with halogen or if R⁵ and R⁶ represent nitro groups, R⁸ and R⁹ can also denote hydrogen and
having the general formula III 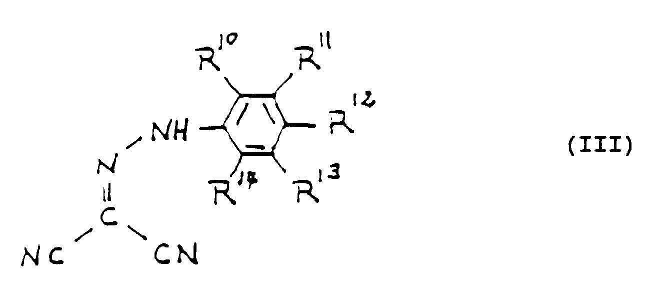 in which
R¹⁰, R¹¹, R¹², R¹³, R¹⁴ are the same or different and each denotes hydrogen, a nitro group, halogen, a cyano group, an alkylsulfonyl group or an alkyl group substituted with halogen,
is used in the phase which is immiscible with the aqueous liquid.

2. Method as claimed in claim 1, wherein a naphthol derivative having the general formula IV 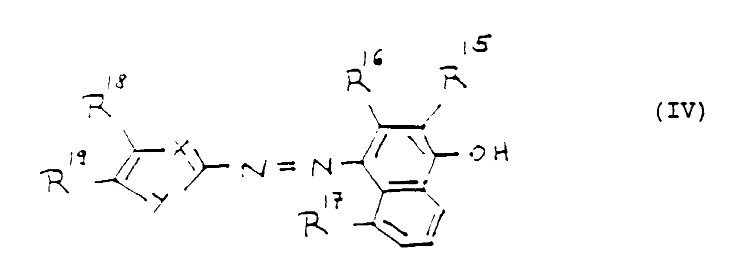 in which
R¹⁵, R¹⁶, R¹⁷ are the same or different and each represents hydrogen, an alkyl or alkoxy group in which at least one of the residues is a (C₈-C₃₀)-alkyl or alkoxy residue,
R¹⁸ is hydrogen or an alkyl group,
R¹⁹ is a nitro group, an alkyl group substituted by halogen, a cyano group, a sulfonamide group or an alkylsulfonyl group,
X is nitrogen or the residue CR²⁰ and
Y is sulphur or the residue CR²¹ = CR²²
in which R²⁰, R²¹, R²² are the same or different and each denotes hydrogen, halogen, a nitro group, a cyano group, an alkyl group or an alkyl group substituted by halogen or an alkylsulfonyl group, is used as the pH indicator.

3. Use of a substance from the group of compounds having the general formula I 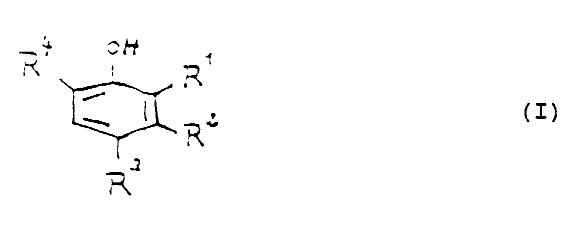 in which
one of the residues R¹ to R⁴ is a residue from the group of alkyl residues, alkoxy residues and aralkyl residues and
the other residues are the same or different and each denotes a nitro group, halogen, a cyano group, an alkylsulfonyl group or an alkyl group substituted with halogen,
having the general formula II 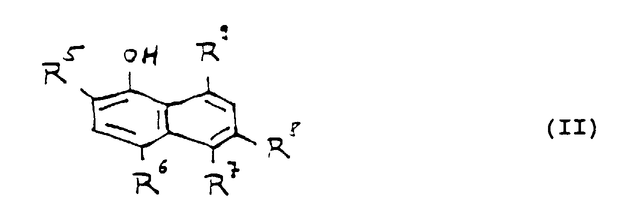 in which
one of the residues R⁵ to R⁹ is a residue from the group of alkyl residues, alkoxy residues and aralkyl residues and
the other residues are the same or different and each denotes a nitro group, halogen, a cyano group, an alkylsulfonyl group or an alkyl group substituted with halogen or if R⁵ and R⁶ represent nitro groups, R⁸ and R⁹ can also denote hydrogen and
having the general formula III 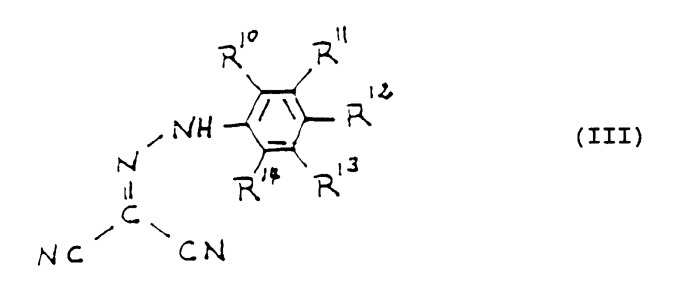 in which
R¹⁰, R¹¹, R¹², R¹³, R¹⁴ are the same or different and each denotes hydrogen, a nitro group, halogen, a cyano group an alkylsulfonyl group or an alkyl group substituted with halogen,
in order to increase the measurement sensitivity in methods for the determination of ions in which the ions pass by means of ionophores into a phase which is immiscible with the aqueous liquid and as a result a pH indicator which is present there undergoes a colour change which is used for the determination of the ions.

4. Agent for the determination of an ion in an aqueous liquid containing an ionophore and a pH indicator in a medium which is immiscible with water, wherein in order to increase the measurement sensitivity it contains a substance from the group of compounds having the general formula I 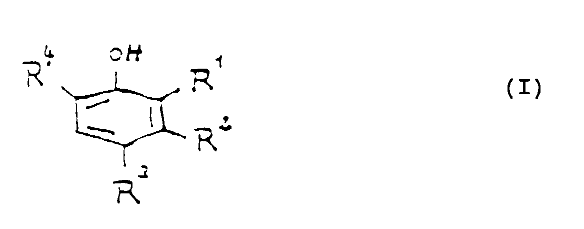 in which
one of the residues R¹ to R⁴ is a residue from the group of alkyl residues, alkoxy residues and aralkyl residues and
the other residues are the same or different and each denotes a nitro group, halogen, a cyano group, an alkylsulfonyl group or an alkyl group substituted with halogen,
having the general formula II 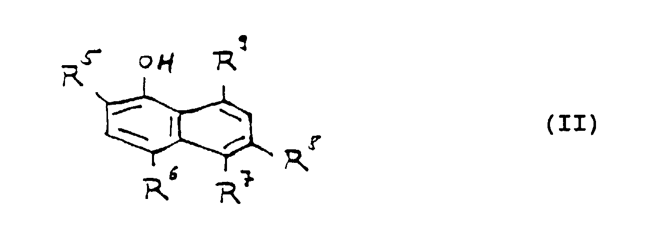 in which
one of the residues R⁵ to R⁹ is a residue from the group of alkyl residues, alkoxy residues and aralkyl residues and
the other residues are the same or different and each denotes a nitro group, halogen, a cyano group, an alkylsulfonyl group or an alkyl group substituted with halogen or if R⁵ and R⁶ denote nitro groups, R⁸ and R⁹ can also denote hydrogen and
having the general formula III 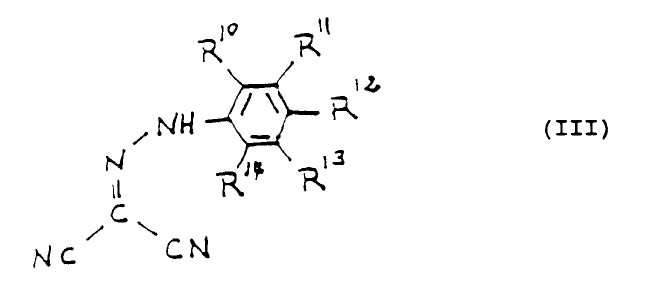 in which
R¹⁰, R¹¹, R¹², R¹³, R¹⁴ are the same or different and each denotes hydrogen, a nitro group, halogen, a cyano group, an alkylsulfonyl group or an alkyl group substituted with halogen.

5. Agent as claimed in claim 4, wherein it contains a naphthol derivative having the general formula IV as the pH indicator 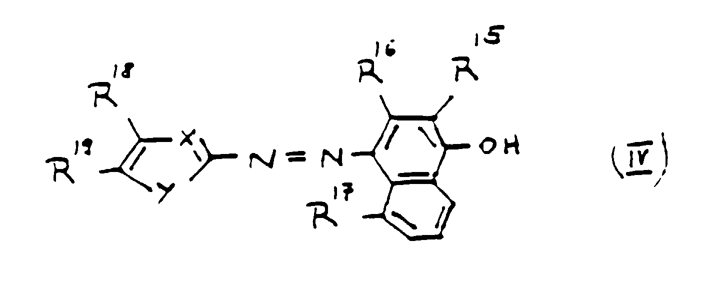 in which
R¹⁵, R¹⁶, R¹⁷ are the same or different and each represents hydrogen, an alkyl or alkoxy group in which at least one of the residues is a (C₈-C₃₀)-alkyl or alkoxy residue,
R¹⁸ is hydrogen or an alkyl group,
R¹⁹ is a nitro group, an alkyl group substituted by halogen, a cyano group, a sulfonamide group or an alkylsulfonyl group,
X is nitrogen or the residue CR²⁰ and
Y is sulphur or the residue CR²¹ = CR²²,
in which R²⁰, R²¹, R²² are the same or different and each denotes hydrogen, halogen, a nitro group, a cyano group, an alkyl group or an alkyl group substituted by halogen or an alkylsulfonyl group.

6. Use of a substance from the group of compounds having the general formula I 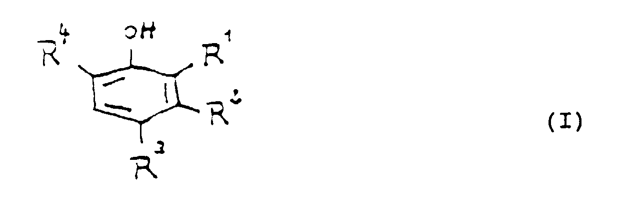 in which
one of the residues R¹ to R⁴ is a residue from the group of alkyl residues, alkoxy residues and aralkyl residues and
the other residues are the same or different and each denotes a nitro group, halogen, a cyano group, an alkylsulfonyl group or an alkyl group substituted with halogen,
having the general formula II 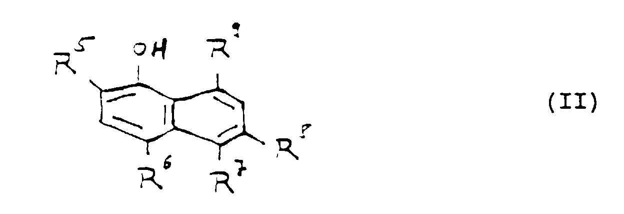 in which
one of the residues R⁵ to R⁹ is a residue from the group of alkyl residues, alkoxy residues and aralkyl residues and
the other residues are the same or different and each denotes a nitro group, halogen, a cyano group, an alkylsulfonyl group or an alkyl group substituted with halogen or if R⁵ and R⁶ represent nitro groups, R⁸ and R⁹ can also denote hydrogen and
having the general formula III 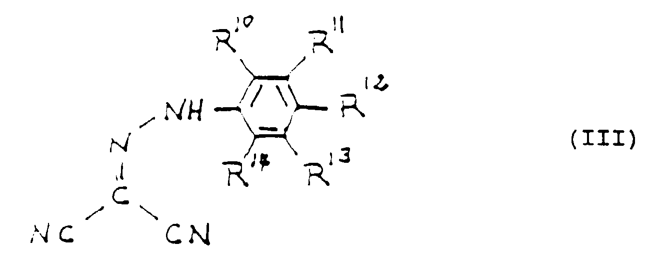 in which
R¹⁰, R¹¹, R¹², R¹³, R¹⁴ are the same or different and each denotes hydrogen, a nitro group, halogen, a cyano group, an alkylsulfonyl group or an alkyl group substituted with halogen,
in order to produce an agent for the determination of an ion which contains an ionophore and a pH indicator in a medium which is immiscible with water.

7. Compound having the general formula II 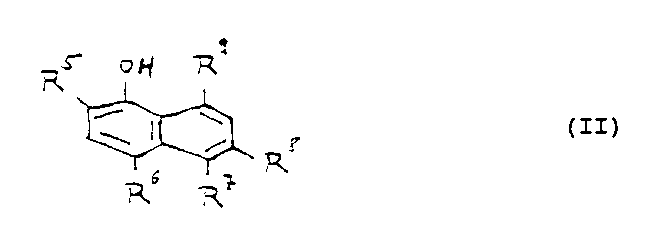 in which
R⁷ is an alkyl, alkoxy or aralkyl residue and the other residues are the same or different and each denotes a nitro group, halogen, a cyano group, an alkylsulfonyl group or an alkyl group substituted with halogen or if R⁵ and R⁶ represent nitro groups, R⁸ and R⁹ can also denote hydrogen.

8. Compound from the group of substances
[(2,3,5,6-tetrafluorophenyl)-hydrazono]propanedinitrile,
[(2-trifluoromethyl-4-nitrophenyl)-hydrazono]-propanedinitrile,
[(2-methanesulfonyl-4-nitrophenyl)-hydrazono]-propanedinitrile,
[(2,4-dinitro-6-cyanophenyl)-hydrazono]propane-dinitrile and
[(3,5-di-(trifluoromethyl)phenyl)-hydrazono]-propanedinitrile.

## Revendications

1. Procédé de détermination d'un ion dans un liquide aqueux, dans lequel l'ion, sous l'effet d'un ionophore, passe dans une phase non miscible avec la phase aqueuse et de ce fait, un indicateur de pH qui se trouve dans cette phase subit un changement de couleur qui est utilisé pour la détermination de l'ion, caractérisé par le fait que pour augmenter la sensibilité de mesure dans la phase non miscible avec la phase aqueuse, on utilise une substance appartenant au groupe des composés de formule générale I 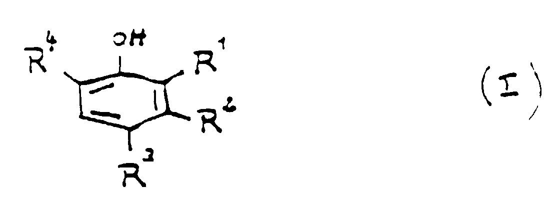 dans laquelle
un des restes R¹ à R⁴ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène,
des composés de formule générale II 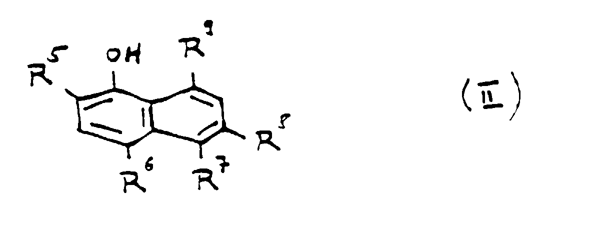 dans laquelle
un des restes R⁵ à R⁹ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène ou, lorsque R⁵ et R⁶ représentent un groupe nitro, R⁸ et R⁹ peuvent également représenter un atome d'hydrogène, et
des composés de formule générale III 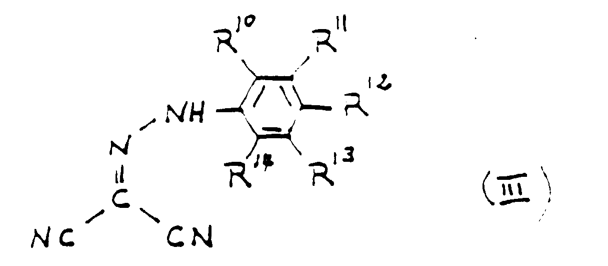 dans laquelle
R¹⁰, R¹¹, R¹², R¹³ et R¹⁴ sont identiques ou différents et représentent chacun un atome d'hydrogène, un groupe nitro, halogéno, cyano. alkylsulfonyle ou un groupe alkyle substitué par un halogène.

2. Procédé selon la revendication 1, caractérisé en ce que, en tant qu'indicateur de pH, on utilise un dérivé de naphtol de formule générale IV 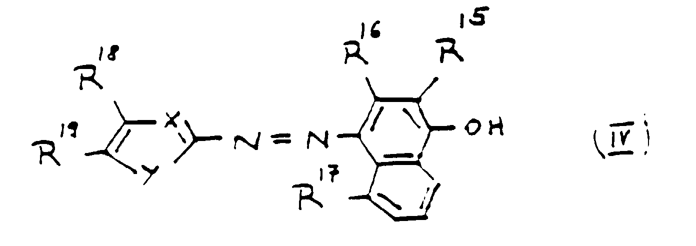 dans laquelle
R¹⁵, R¹⁶, R¹⁷ sont identiques ou différents et représentent chacun un atome d'hydrogène, un groupe alkyle ou alcoxy, au moins un des restes représentant un groupe alkyle ou alcoxy en C₈-C₃₀,
R¹⁸ représente un atome d'hydrogène ou un groupe alkyle,
R¹⁹ représente un groupe nitro, un groupe alkyle substitué par un halogène, un groupe cyano, sulfonamido ou alkylsulfonyle,
X représente un atome d'azote ou le reste CR²⁰ et
Y représente un atome de soufre ou le reste CR²¹=CR²²,
où R²⁰, R²¹, R²² sont identiques ou différents et représentent chacun un atome d'hydrogène, un atome d'halogène, un groupe nitro, cyano, alkyle ou alkyle substitué par un halogène ou un groupe alkylsulfonyle.

3. Utilisation d'une substance du groupe des composés de formule générale I 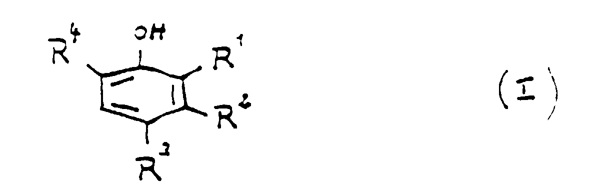 dans laquelle
un des restes R¹ à R⁴ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène,
des composés de formule générale II 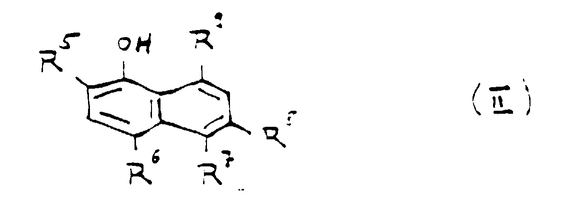 dans laquelle
un des restes R⁵ à R⁹ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène ou, lorsque R⁵ et R⁶ représentent un groupe nitro, R⁸ et R⁹ peuvent également représenter un atome d'hydrogène, et
des composés de formule générale III 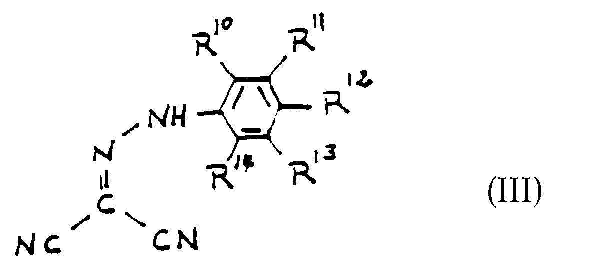 dans laquelle
R¹⁰, R¹¹, R¹², R¹³ et R¹⁴ sont identiques ou différents et représentent chacun un atome d'hydrogène, un groupe nitro, halogéno, cyano, alkylsulfonyle ou un groupe alkyle substitué par un halogène,
pour augmenter la sensibilité de mesure dans des procédés de détermination d'ions dans lesquels les ions, sous l'effet d'un ionophore, passent dans une phase non miscible avec la phase aqueuse et de ce fait, un indicateur de pH qui se trouve dans cette phase subit un changement de couleur qui est utilisé pour la détermination de l'ion.

4. Agent destiné à la détermination d'un ion dans un liquide aqueux, contenant un ionophore et un indicateur de pH dans un milieu non miscible à l'eau, caractérisé en ce que, pour augmenter la sensibilité de mesure, on utilise une substance appartenant au groupe des composés de formule générale I 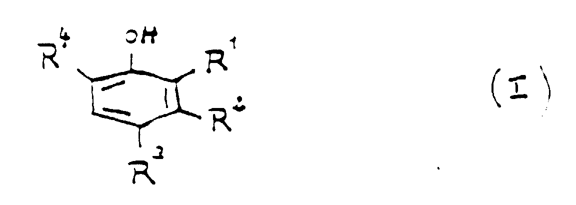 dans laquelle
un des restes R¹ à R⁴ représente un reste choisi dans le groupe formé par les restes alkyle, alcoxy et aralkyle et
les autres restes sont identiques ou différents et représentent chacun un groupe nitro, halogeno, cyano, alkylsulfonyle ou un groupe alkyle substitué par un halogène,
des composés de formule générale II 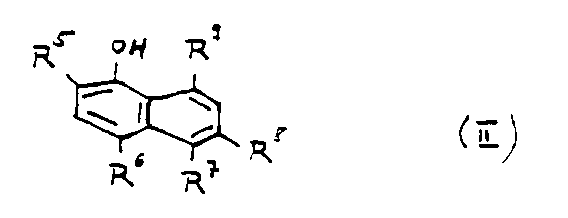 dans laquelle
un des restes R⁵ à R⁹ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène ou, lorsque R⁵ et R⁶ représentent un groupe nitro, R⁸ et R⁹ peuvent également représenter un atome d'hydrogène, et
des composés de formule générale III 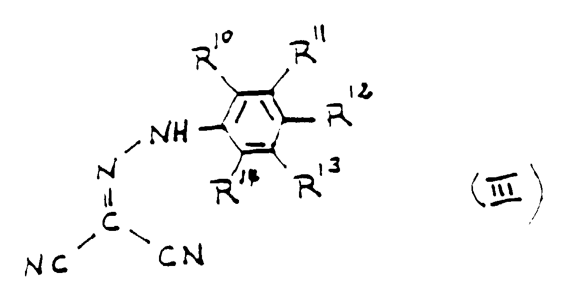 dans laquelle
R¹⁰, R¹¹, R¹², R¹³ et R¹⁴ sont identiques ou différents et représentent chacun un atome d'hydrogène, un groupe nitro, halogéno, cyano, alkylsulfonyle ou un groupe alkyle substitué par un halogène.

5. Agent selon la revendication 4, caractérisé en ce que, en tant qu'indicateur de pH, il contient un dérivé de naphtol de formule générale IV 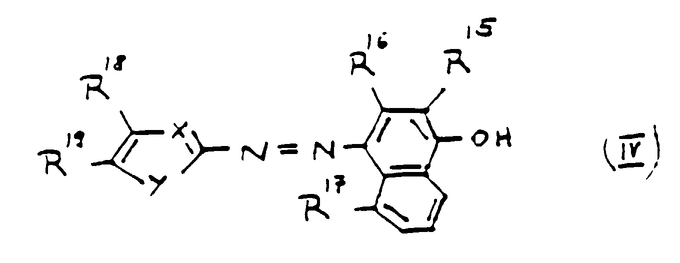 dans laquelle
R¹⁵, R¹⁶, R¹⁷ sont identiques ou différents et représentent chacun un atome d'hydrogène, un groupe alkyle ou alcoxy, au moins un des restes représentant un groupe alkyle ou alcoxy en C₈-C₃₀,
R¹⁸ représente un atome d'hydrogène ou un groupe alkyle,
R¹⁹ représente un groupe nitro, un groupe alkyle substitué par un halogène, un groupe cyano, sulfonamido ou alkylsulfonyle,
X représente un atome d'azote ou le reste CR²⁰ et
Y représente un atome de soufre ou le reste CR²¹=CR²²,
où R²⁰, R²¹, R²² sont identiques ou différents et représentent chacun un atome d'hydrogène, un atome d'halogène, un groupe nitro, cyano, alkyle ou alkyle substitué par un halogène ou un groupe alkylsulfonyle.

6. Utilisation d'une substance du groupe des composés de formule générale I 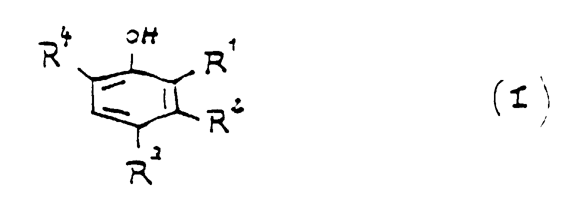 dans laquelle
un des restes R¹ à R⁴ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe allyle substitué par un halogène,
des composés de formule générale II 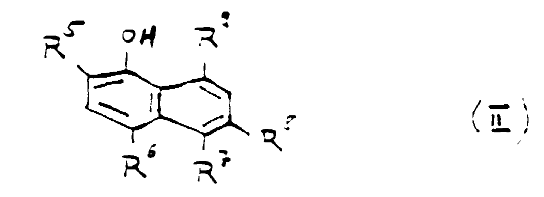 dans laquelle
un des restes R⁵ à R⁹ représente un reste choisi dans le groupe formé par les restes alkyle, alcoxy et aralkyle et
les autres restes sont identiques ou différents et représentent chacun un groupe nitro, halogéno, cyano, alkylsulfonyle ou un groupe alkyle substitué par un halogène ou, lorsque R⁵ et R⁶ représentent un groupe nitro, R⁸ et R⁹ peuvent également représenter un atome d'hydrogène, et
des composés de formule générale III 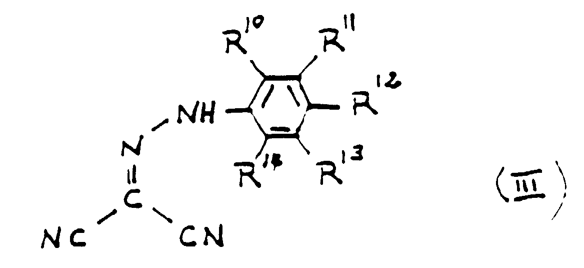 dans laquelle
R¹⁰, R¹¹, R¹², R¹³ et R¹⁴ sont identiques ou différents et représentent chacun un atome d'hydrogène, un groupe nitro, halogéno, cyano, alkylsulfonyle ou un groupe alkyle substitué par un halogène,
pour la préparation d'un agent, destiné à la détermination d'un ion, qui contient un ionophore et un indicateur de pH dans un milieu non miscible à l'eau.

7. Composé de formule générale II 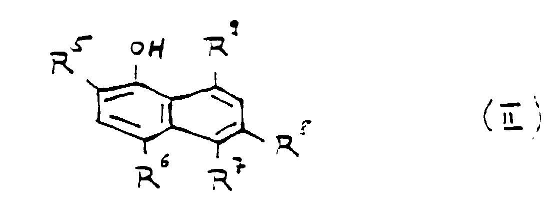 dans laquelle
R⁷ représente un groupe alkyle, alcoxy ou aralkyle et les autres restes sont identiques ou différents et représentent chacun un groupe nitro, halogéno, cyano, alkylsulfonyle ou alkyle substitué par un atome d'halogène, ou lorsque R⁵ et R⁶ représentent un groupe nitro, R⁸ et R⁹ peuvent également représenter un atome d'hydrogène.

8. Composé appartenant au groupe des substances
[(2,3,5,6-tétrafluorophényl)-hydrazono]propanedinitrile,
[(2-trifluorométhyl-4-nitrophényl)-hydrazono]propanedinitrile.
[(2-méthanesulfonyle-4-nitrophényl)-hydrazono]propanedinitrile.
[(2,4-dinitro-6-cyanophényl)-hydrazono]propanedinitrile et
[(3,5-di(trifluorométhyl)phényl)-hydrazono]propanedinitrile.
